(19) Europäisches Patentamt
European Patent Office
Office européen des brevets

(11) **EP 2 486 851 B1**

(12) **EUROPEAN PATENT SPECIFICATION**

(45) Date of publication and mention
of the grant of the patent:
**27.09.2023   Bulletin 2023/39**

(21) Application number: **12000442.9**

(22) Date of filing: **25.01.2012**

(51) International Patent Classification (IPC):
**A61B 5/145** $^{(2006.01)}$      **G16H 40/63** $^{(2018.01)}$
**G16H 50/70** $^{(2018.01)}$

(52) Cooperative Patent Classification (CPC):
**A61B 5/14532; A61B 5/7275; A61B 5/743;**
**G16H 40/63; G16H 50/70;** G16H 20/10;
G16H 20/30; G16H 20/60; G16H 20/70

(54) **Display for biological values**

Anzeige für biologische Werte

Affichage pour des valeurs biologiques

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **31.01.2011   US 201113017526**

(43) Date of publication of application:
**15.08.2012   Bulletin 2012/33**

(73) Proprietors:
• **Roche Diabetes Care GmbH**
**68305 Mannheim (DE)**
Designated Contracting States:
**DE**
• **F. Hoffmann-La Roche AG**
**4070 Basel (CH)**
Designated Contracting States:
**AL AT BE BG CH CY CZ DK EE ES FI FR GB GR**
**HR HU IE IS IT LI LT LU LV MC MK MT NL NO PL**
**PT RO RS SE SI SK SM TR**

(72) Inventors:
• **Amann-Zalan, Ildiko**
**69469 Weinheim (DE)**
• **Galley, Paul**
**Cumberland**
**IN 46229 (US)**
• **Greenburg, Alan**
**Indianapolis**
**IN 46256-1181 (US)**
• **Rasch-Menges, Juergen**
**68723 Schwetzingen (DE)**

• **Soni, Abhishek**
**Indianapolis**
**IN 46280 (US)**
• **Thukral, Ajay**
**Fishers**
**IN 46037 (US)**
• **Weinert, Stefan**
**Pendleton**
**IN 46064 (US)**

(74) Representative: **Bittner, Thomas L.**
**Boehmert & Boehmert**
**Anwaltspartnerschaft mbB**
**Pettenkoferstrasse 22**
**80336 München (DE)**

(56) References cited:
EP-A2- 0 483 595        US-A- 5 822 715
US-A1- 2008 125 636     US-A1- 2008 214 941
US-A1- 2009 150 186     US-A1- 2010 330 598
US-A1- 2010 331 627     US-A1- 2011 015 511

• STREJA ET AL: "Optimizing diabetes
management through glucose profiling: A
case-based approach", PRIMARY CARE
DIABETES, ELSEVIER, AMSTERDAM, NL, vol. 2,
no. 4, 1 December 2008 (2008-12-01), pages
167-173, XP025696310, ISSN: 1751-9918, DOI:
10.1016/J.PCD.2008.06.004 [retrieved on
2008-09-10]

## Description

**Technical Field**

The disclosure relates to physiological monitoring, and in particular, to a methods and displays for providing estimated and predicted biological values from biological measurements.

**Background**

For monitoring glycemia, the American Diabetes Association (ADA) recommends the hemoglobin A1C test, hereinafter referred to HbA1C. Health care providers (HCPs) use HbA1C as a surrogate marker to evaluate a patient's glycemia over a previous 2 to 3 month period and as a target parameter by which to treat patients. For example, the HbA1C value, which can be presented as a percentage of glycated hemoglobin or in international standardized units mmol/mol (by International Federation of Clinical Chemistry, IFCC), is needed by the HCP when deciding or recommending a change to a patient's therapy. Therapy modification may include a change to, an addition to, or a switch in insulin therapy, oral medication, nutrition, physical activity, or combinations thereof in order to regulate a patient's glucose with the goal of improving a patient's HbA1C value. For a high quality determination (i.e., coefficient of variation (CV) < 3%) of the HbA1C value, HbA1C assays are the norm in which blood samples are tested for the extent of glycation of hemoglobin by use of laboratory devices such as, for example, a D-10 analyzer from Bio-Rad Laboratories, or a G-7 analyzer from Tosoh Bioscience, Inc. For an approximated assessment of glycemia, HCPs alternatively use blood glucose (bG) values to determine an average glucose value, and then interpret the results to derive an estimated HbA1C value from spot monitoring blood glucose (SMBG) values. However, the estimated HbA1C value so obtained by such a method, in general, is poor in quality (i.e., CV >5%).

Other methods of solving a true mean bG value and estimating HbA1C value have been based on both the SMBG data collected during various clinical trials and the relationships derived there from. For example, many such methods use SMBG data to develop prediction models based on statistical methods. Other methods consider weighted bG value schemes with additional predicators, such as a previous HbA1C value, to determine an estimated HbA1C value using a noted study relationship. While still other methods further include transforming a bG value and then using the transformed bG value to determine the estimated HbA1C value, However, such methods have the following potential issues: model parameters typically needs retuning, correlation is still generally poor (i.e., CV>5%), the standard errors are typically large, and adjustments to account for lifestyle related variations are not made such that any such reported patient specific solution is not specific enough to account for lifestyle related variations.

It is to be appreciated that one of the key limiting factors to finding a good generic algorithm which provides an accurate HbA1C estimation (i.e., determining the current HbA1C value) or prediction (i.e., determining the future HbA1C value) is the difficulty in obtaining comprehensive and detail (frequently sampled) blood glucose data under various conditions. For instance, studies having data sets based on continuous blood glucose monitoring, although providing dense data are typically conducted on relatively smaller population sizes and with durations that are relatively shorter in time than studies with SMBG data sets. With SMBG, on the other hand, there is a practical limitation of how many measurements can be collected, Since bG varies during the day, due to many factors such as physical activity, meal response, drug response (such as oral drugs or insulin) and stress and so forth, it is not possible to get an accurate picture of a glucose excursion by just a few daily measurements. This means that the SMBG data sets (i.e., time-interval based data sets) often fail to capture true bG variation of the patient with diabetes (PwD). The implication is that the resulting prediction models are normally then very study specific. Such prediction models therefore can neither be extended to account for other variables not addressed by the study(-ies) which they were based on nor used in an alternate situation to make predictions without the need for an additional clinical trial to validate such model extensions. Furthermore, as such methods fail to account for the context associated with bG measurements or in other words, to account for influence(s) of events such as carbohydrate ingestion, physical activity, insulin therapy, oral drug therapy, and so forth, such methods are generally unsuitable for determining an estimated HbA1C value of good quality (i.e., CV <3%) for a patient specific lifestyle. The lack of context associated with measurements can also limit the application of results when studying other glycemic excursion or non glycemic excursion factors (e.g., lipid profiles, insulin concentration profile, heart rate profile assuming availability of spot/continuous monitoring of the respective parameter). Finally, such methods fail to provide the estimated parameter, such as the estimated HbA1C values (or other parameters such as mean glucose, weighted glucose, fructosamine, biomarkers for various lipid levels, etc,) in a manner that can allow one to assess the relative impact of various components on a patient's overall HbA1C in a manner that would provide a quick evaluation of an implemented therapy. US 2008125636 discloses embodiments which provide methods, apparatuses, and systems associated with detecting, analyzing, and/or displaying historical glucose levels and/or trends in a body. US5822715 discloses a diabetes management system for predicting a future blood glucose value of a patient and for recommending a corrective action to the patient when the future blood glucose value lies outside of a target range. The system includes a patient-operated apparatus for measuring blood glucose values and for storing data relating to insulin doses administered to the patient. The apparatus predicts the patient's future blood glucose value based upon the patient's current blood glucose value, the fraction of insulin action remaining from the insulin doses, and the patient's insulin sensitivity. The apparatus also determines the corrective action for the patient when the predicted blood glucose value lies outside of a target range. The system also includes a physician computer in communication with the apparatus for receiving the blood glucose values and insulin dose data and for calculating an adjusted insulin sensitivity for use in subsequent predictions.

EP0483595 discloses an automated diabetes data interpretation system is provided which combines symbolic and numeric computing approaches in order to identify and highlight key clinical findings in the patient's self-recorded diabetes data. The patent data, including blood glucose levels and insulin dosage levels, recorded by a diabetic patient over a period of time by means of a glucose meter or the like, is initially downloaded into a central processing system such as a personal computer.; The accepted diabetes data is subsequently processed to (a) identify insulin dosage regimens corresponding to predefined significant changes in insulin dosage which are found to be sustained for at least a predefined segment of the overall data collection period, (b) identify statistically significant changes in blood glucose levels resulting across adjacent ones of the identified insulin regimen periods, and (c) identify clinically significant changes in blood glucose levels from within the identified statistically significant glucose level changes. The results of the diabetes data processing are generated in the form of a comprehensive yet easily understandable data interpretation report highlighting the processing results, including details pertaining to the identified insulin regimens and the associated clinically significant changes in glucose levels.

US2008 214841 discloses A blood pressure self-monitoring device includes an alarm generator to remind the patient to carry out blood pressure measurements according to a predetermined schedule. The device performs a specific phase of measuring blood pressure according to a clinically validated criterion, and calculates an accurate blood pressure reading on the basis of plural measurement values. The device also has a therapeutic decision mode, which can monitor blood pressure for assessing the effect of antihypertensive drug treatment. In the extended mode, the alarm generator works according to a predetermined daily measurement to remind the patient to take a blood pressure measurement following drug intake. Also during the treatment phase, the antihypertensive drug may affect the probability of irregular heartbeat, which can be a reference index for assessing the types and doses of treated antihypertensive drug. The device can also use a selectable switch to operate as a regular home blood pressure monitor.

Primary Care Diabetes, 20081201 ELSEVIER, AMSTERDAM, NL - ISSN 1751-9918 discloses that postprandial self-monitoring of blood glucose values are mor tightly correlated to HbA1C than are fasting values.

US 2010/330598 A1 discloses the collection of biological measurements together with their associated context according to a sampling schema and the flagging of the data for non-adherence to the sampling schema.

## Summary

In one embodiment not covered by the claims, a method for providing an estimated or predicted biological values in a sectioned display to assess the relative impact of a set of variables is provided. The method includes collecting biological measurements, grouping the biological measurements based on the set of variables, and evaluating the biological measurements to determine grouped estimated biological values or grouped predicted biological values. The method further provides the grouped estimated biological values or grouped predicted biological values within a plurality of sections in the sectioned display, wherein the plurality of sections correspond to the set of variables. The data is analyzed for adherence to a testing protocol and the lack of adherence if the measurements collected are determined to not adhere is flagged. The measurements are collected around a structured medication schema, and the biological measurements are grouped to appreciate the effect each medication schema has on the estimated or predicted biological values so that the relative impact various medications have is understandable.

A sectioned display device according to the invention is defined in claim 1.

These and other advantages and features disclosed herein will be made more apparent from the description, drawings and claims that follow.

## Brief Description of the Drawings

The following detailed description of the embodiments of the present invention can be best understood when read in conjunction with the following drawings, where like structure is indicated with like reference numerals, and in which:

FIG. 1 depicts a tabulated dataset from simulation of a Mortensen Model based system with a simplification according to the present disclosure;

FIG. 2 depicts graphically HbA1C values generated by simulation which are plotted against mean bG values;

FIG. 3 depicts graphically a disturbance model;

FIG. 4 depicts graphically results obtained from simulation that show true mean bG is linearly related to HbAlC, whereby both positive and negative glycemic variations are illustrated;

FIG. 5 depicts graphically a normal daily lifestyle pattern of an individual for a modal day that consists of an overnight period and 3 meal types: breakfast, lunch and supper;

FIG. 6 depicts in block diagram meal selections from a glycemic excursion perspective categorized per meal type, meal amount, and meal speed;

FIG. 7 depicts graphically a grouping of glucose sections by event type to characterize and quantify the sections in order to help identify parameters that provided a high correlation ratio;

FIG. 8 depicts graphically 4 weighting schemes considered in developing a prediction model for HbA1C according to the present disclosure;

FIG. 9 depicts graphically a strong linear relationship between HbA1C and post prandial bG measurement when $t \geq 150$ minutes;

FIG. 10 depicts graphically quality of estimated HbA1C for various values of a Window Center (WinCen) and a Window Size (WinSize) for lifestyle context plotted by R-squared values;

FIG. 11 depicts graphically quality of estimated HbA1C for various values of a Window Center (WinCen) and a Window Size (WinSize) for lifestyle context plotted by mean squared errors;

FIG. 12 depicts graphically a comparison between daily lifestyle weighting and no daily lifestyle weighting and showing that daily life style weighting produces lower mean squared error;

FIG. 13 depicts graphically impact of visitation period (nDays) and number of sample (nSamples) for a WinCen of 190 minutes and a WinSize of 50 minutes;

FIG. 14 depicts graphically a sampling ratio plotted against R-squared values;

FIGS. 15A-E each depict graphically a sampling schema for sampled bG data being regressed and plotted by HbA1C %, and showing that the parameters for each linear regression are in close proximity to each other;

FIGS. 16A-E each depict graphically a sampling schema for sampled bG data being regressed and plotted by HbA1C % with a prediction line (center line) and a 95% confidence interval (CI) boundaries (above and below curves) shown in the subplots;

FIG. 17 depicts in block diagram a processor based system according to one or more embodiments shown or described herein;

FIG. 18 is a flow diagram for processing data according to one or more embodiments shown or described herein;

FIG. 19 is another flow diagram for processing data according to one or more embodiments shown or described herein;

FIG. 20 is a flow diagram of a delivery method for providing grouped estimated HbA1C values in a sectioned display according to one or more embodiments shown or described herein;

FIG. 21 is an exemplary visualization of grouping biological measurements in preparation for providing grouped values in a sectioned display according to one or more embodiments shown or described herein;

FIG. 22 is a first exemplary sectioned display according to one or more embodiments shown or described herein;

FIG. 23 is a second exemplary sectioned display according to one or more embodiments shown or described herein;

FIG. 24 is a third exemplary sectioned display according to one or more embodiments shown or described herein;

FIG. 25 is a fourth exemplary sectioned display according to one or more embodiments shown or described herein;

FIG. 26 is a fifth exemplary sectioned display according to one or more embodiments shown or described herein;

FIG. 27 is a sixth exemplary sectioned display according to one or more embodiments shown or described herein;

FIG. 28 is a seventh exemplary sectioned display according to one or more embodiments shown or described herein;

FIG. 29 is an eighth exemplary sectioned display according to one or more embodiments shown or described herein;

FIG. 30 is a ninth exemplary sectioned display according to one or more embodiments shown or described herein;

FIG. 31 is an exemplary textual screen for prompting and conveying detailed information regarding the biological measurements and/or the estimated or predicted biological values according to one or more embodiments shown or described herein; and

FIG. 32 is a flow diagram of a selective display method according to one or more embodiments shown or described herein.

## Detailed Description

It is to be appreciated that embodiments of the present disclosure enhance existing software and/or hardware that retrieves and processes biological measurements such as blood glucose (bG) data. The embodiments of the disclosure can be directly incorporated into existing home glucose monitors, or used for the enhancement of software that retrieves and processes biological measurements (e.g., bG data), by introducing a method for delivering estimated biological values (e.g., estimated glycated hemoglobin (HbA1C) values) of good quality from structured spot biological measurements having a coefficient of variation (CV) of less than 5% in one embodiment, and less than 3% in a preferably embodiment.

In the sections to follow, a discussion is made first to the exemplary approach used to derive the equations for providing the estimated true mean blood glucose (bG) value and the estimated glycated hemoglobin (HbA1C) value from structured spot measurements of blood glucose (i.e., bG data) collected, as per a measurement schema according to the present disclosure. It is to be appreciated that the measurement schema according to the present disclosure assumes that the PwD maintains a repeatable average behavior, whereby collection exceptions (i.e., missed testing times) are managed by the algorithm on the estimated HbA1C value. It is further to be appreciated that the utility of providing an estimated HbA1C value that demonstrates continuous blood glucose monitoring will provide a fairly accurate idea of the overall level of glycemia of the PwD, as compared to the uncertainty associated with estimating glycemia and its variation based only on spot monitoring. Additionally, certain HbA1C values have been linked to various disease states, and thus having a good estimated HbA1c value between laboratory based assays values can help identify much earlier a patient's potential risk associated to long term complications, such as micro-vascular (retinopathy, neuropathy, nephropathy) disease complications. Furthermore, providing an assessment of overall glycemia via an estimated HbA1C value of good quality can empower the PwD to manage better his/her diabetes. Alternatively, using a shorter window the algorithm can provide predicted HbA1c which allows PwD and HCP impact of current lifestyle on future HbA1c. A discussion of the methodology used to provide the estimated true mean blood glucose value, estimated glycated hemoglobin (HbA1C) value and other estimated and predicted biological values from collected biological measurements according to the present disclosure now follows.

*Kinetics of glycation of hemoglobin*

Glycation is a non-enzymatic chemical reaction wherein the glucose molecules bind with the amino acid groups of the proteins. Of the many glycated proteins hemoglobin A1C is fairly stable and one of the dominant forms of glycohemoglobin. Synthesis of HbA1C is primarily a condensation of hexose with the hemoglobin structure to form an unstable intermediate Schiff base adduct, or aldimine, followed by the Amadori rearrangement to form the stable ketoamine adduct, HbA1c. The kinetics of the glycation of hemoglobin to surrounding glucose concentration can be modeled by three differential equation, Equations (1)-(3), which are disclosed more fully by the publication of Mortensen, H. B.; "Glycated hemoglobin. Reaction and biokinetic studies. Clinical application of hemoglobin Alc in the assessment of metabolic control in children with diabetes mellitus," Danish medical bulletin (1985), 32(6), pp. 309-328. The model according to Equations (1)-(3), is referred herein as the Mortensen Model.

*Mortensen Model*

$$\frac{dH_{bA}}{dt} = -k_{12}H_{bA}G + k_{21}H_{bA1d} \tag{1},$$

$$\frac{dH_{bA1d}}{dt} = k_{12}H_{bA}G - \left(k_{21} + k_{23}\right)H_{bA1d} + k_{32}H_{bA1c} \tag{2},$$

and

$$\frac{dH_{bA1c}}{dt} = k_{23}H_{bA1d} - k_{32}H_{bA1c} \tag{3}.$$

In the Mortensen model, the term $H_{bA}$ represents the sub-pool of erythrocytes of same age, whereby the pool consists of cohorts of erythrocytes of varying age. The behavior of each cohort is represented by a corresponding set of Equations (1)-(3). From the Mortensen publication, the k parameters used in the model are known as follows: $k_{12}$ = 5.76 mmol/l/min; $k_{21}$ = 0.006 /min; $k_{23}$ = 0.000852 /min; and $k_{32}$ = 0.000102 /min. Next, to test the utility of the Mortensen model in helping to generate a basic relation between HbA IC and bG, glycation simulations were run on simulated data for meal related bG excursions which is discussed hereafter.

*Glycation simulation setup*

Meal related bG excursions were evaluated first using simple mathematical formulas, whereby simulated data helped to generate a basic relation between HbA1C and bG. It is to be appreciated that the glycation of erythrocytes is a continuous process. However, the erythrocytes have finite lifespan of approximately 120 days. Depending on problem needs one can use other lifespan values such as ranging more or less from 90 ~ 120 days to cover different population groups and/or physiological conditions. This means that in addition to the glycation, erythrocytes are continuously being added and removed from the glycation process. As the aged erythrocytes are replaced, the state of glycation of all the cells has to be managed. From a simulation perspective, instead of using Equations (1)-(3) for each cell, a simplification was done by grouping cells into cohorts of equally aged cells. In particular for the glycation simulation setup, n numbers of cohorts of erythrocytes were considered, whereby each of the cohorts was described by the set of the 3 differential equations (Equations (1)-(3)). Each cohort is assumed to have a life span of n days. When a cohort's maximum age is reached, a new cohort replaces it. The simulation handled this by resetting the 3-states of the oldest cohort (i.e. when the age of the cohort reaches its life-duration of 120 days) to the state of a fresh cohort of erythrocytes with non-glycated hemoglobin. In all, there were *n* sets of differential equations used in the simulation, whereby each set of equations represented a state of the corresponding cohort.

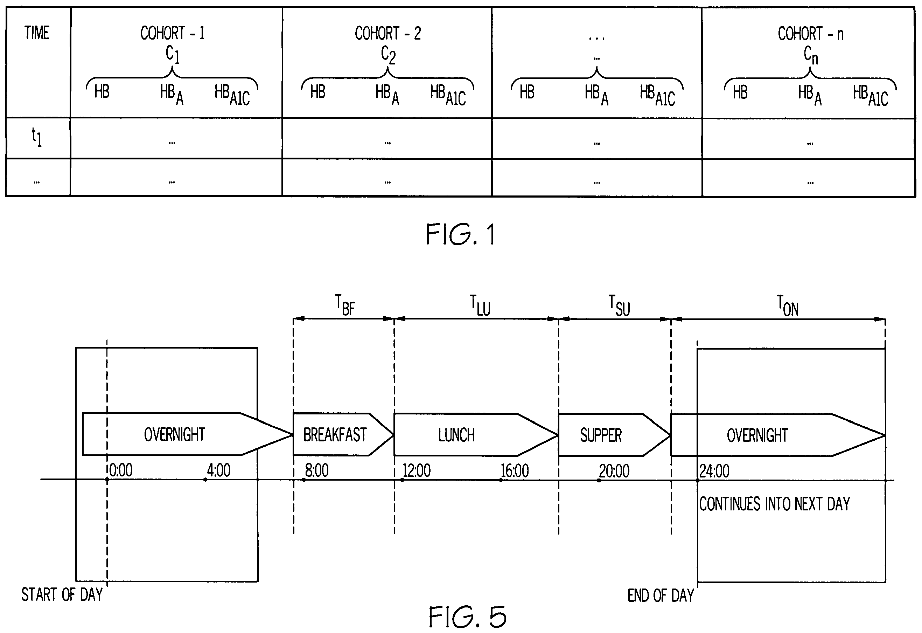

The 3n states were stored in columns as shown schematically in FIG. 1, which represent a tabulated dataset from the simulation of the Mortensen Model system using the above mentioned simplification. At each new time instant, the values for each of the states were recorded in the next new row as a record set, whereby glycation of each cohort at any given time is the value of the 3$^{rd}$ state. The net HbA1C in percentage (%) can be given by summing the HbA1C state for each of the cohort according to Equation (4):

$$HB_{A1C} = 100 \sum_{i=3,9\ldots}^{n} x_i \qquad (4),$$

where the summation counter i is the column corresponding to the $HB_{A1C}$ state. Using Equations (1)-(4), HbA1C can be simulated for an arbitrary bG profile. The true mean blood glucose value, $\overline{bG}$ can be thus given by Equation 5:

$$\overline{bG} = \frac{AUC}{Duration} \qquad (5),$$

where AUC is the area under a continuous bG excursion curve. However, when bG measurements are sparse and non-continuous, as in the case of spot bG measurements, then it is to be appreciated that Equation 5 is no longer valid. Accordingly, a new relationship was derived in order to estimate the true mean bG as follows.

*Simulated Cases*

Under the above mentioned idealized setup, the relationship between periodic glucose profiles and corresponding HbA1C values was then examined for deriving useful insights and relationships. Specifically, two profiles were examined: (1) Sinusoidal glucose profile with offset (Equation (6)); and (2) Gamma function profile with offset (Equation (8)). These functions can be seen as representative of the meal event with post-prandial glucose behavior for varying levels of control, whereby constant glucose is a special case of both of the functions. For the sinusoidal glucose profile, Equation (6) is defined as:

$$bG = bG_{const} + \frac{A}{2}\left(1 - \cos\left(\frac{2\pi}{T}t\right)\right) \qquad (6),$$

where, $bG_{const}$ provides the steady state offset and

$$\frac{A}{2}\left(1-\cos\left(\frac{2\pi}{T}t\right)\right)$$

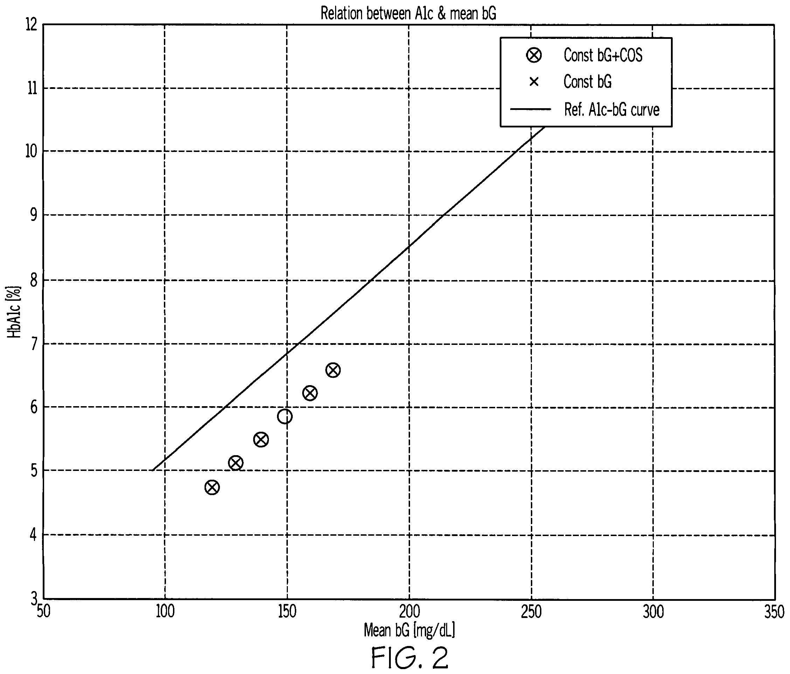

is the cosine curve with amplitude A and period T. FIG. 2 shows the HbA I C values generated by the simulation plotted against the mean bG values. The results in FIG. 2 show that the true mean bG for the continuous glucose profile and simulated HbA1C is approximately linear as shown by the 'o' symbols. It also shows that HbA1C obtained by a constant bG and that from oscillating bG are approximately identical if they have the same mean bG value. If comparing the HbA1C resulting from two sinusoidal inputs, which are identical except for the frequency, the HbA1C from the slower varying signal will have comparatively higher glycation rate. The rate of signal has an effect but under conditions of interest it is small. The solid curve shows a known relationship between HbA1C and mean bG and is used as a reference.

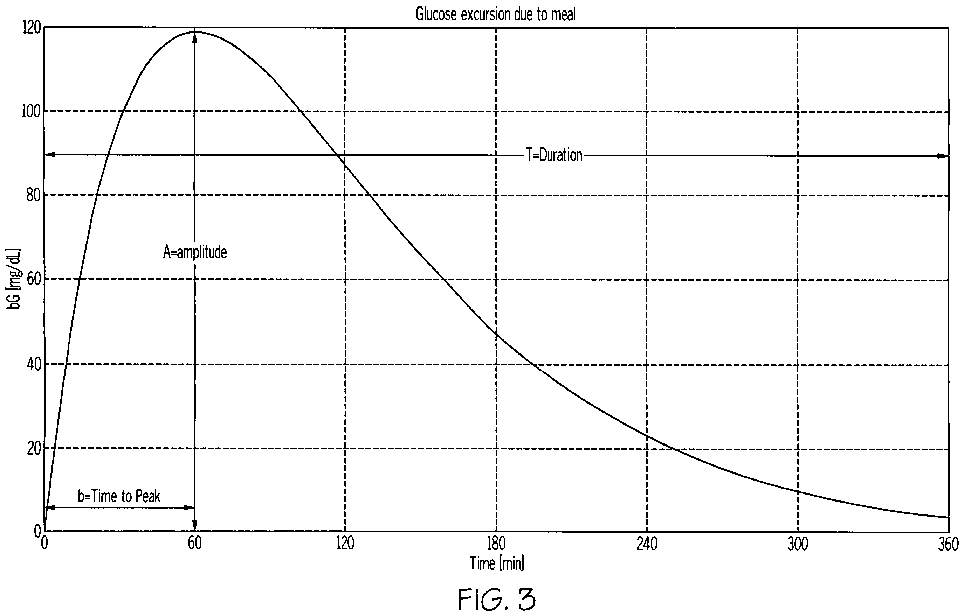

The disturbance model (in which the gamma function was used to model disturbance) is shown by FIG. 3, and is described by the function defined by Equation (7):

$$f(t) = \frac{t^{\alpha-1}e^{-t/\beta}}{\beta^{\alpha}\Gamma(\alpha)}, t \geq 0 \qquad (7).$$

In this simulation embodiment, the Gamma function was used to represent the post meal glucose excursion. Grossly approximated, the model shows a two (2) compartment model of glucose in a post prandial state. It has been used primarily to understand the impact of glucose varying from the aspects of different rates of postprandial rise, decay and magnitude of an excursion. The parameters $\alpha$ and $\beta$ approximately represent the number of compartments and time to peak. In fact, if $\alpha$ is set to 2, a 2nd order compartment model is considered with a time constant for both compartments equal to $\beta$ (2nd order system with repeated poles). Therefore, the above function according to Equation (7) simplifies to:

$$f(t) = \frac{te^{-t/\beta}}{\beta^2},$$

, $t \geq 0$. The peak value of the function $f(t)$ is reached when t=$\beta$. The peak value is then $\frac{1}{e\beta}$ . Therefore, the glucose excursion used herein can be defined by Equation 8:

$$bG(t) = bG_{const} + \frac{Ae}{\beta}te^{-t/\beta}, t \geq 0 \qquad (8),$$

where $A$ is the peak bG value with respect to $bG_{const}$.

The response of HbA1C to glucose excursions for various time to peak and peak values was then studied both analytically and in simulation. The Gamma functions for the various combinations of the parameters studied are listed in Table 1.

**Table 1: Parameter settings for Gamma function**

| Parameter | Values |
|---|---|
| Constant, $bG_{const}$ [mg/dL] | 80, 100, 120, 140 |
| Amplitude, A [mg/dL] | Positive (+ve) glucose push<br>0, 40, 60, 80, 100, 120, 140, 180, 300 |
| | Negative (-ve) glucose push<br>0, 40, 60, 80 |
| Periodicity [hour] | 4, 6, 8, 12, 24 |

(continued)

| Parameter | Values |
|---|---|
| Time to peak, $\beta$ [minute] | 30, 60, 90, 120 |

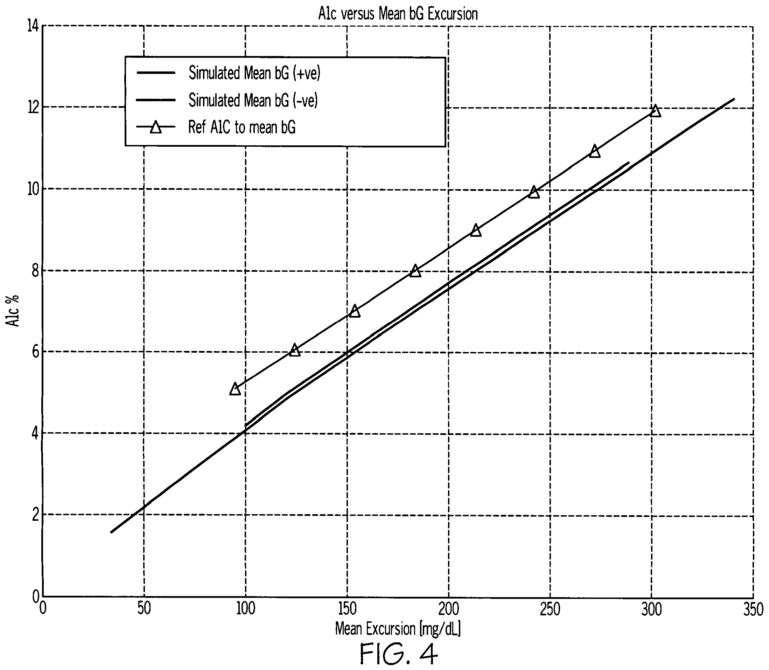

The results obtained from simulation show that the true mean bG is linearly related to HbA1C. This linear relationship is shown by FIG. 4, whereby both positive and negative glycemic variations are illustrated. The solid line above 4% HbA1C is for positive glycemic variation with respect to a constant 100 mg/dL signal and the dashed line below 4% HbA1C is for the negative glycemic variation. In order to provide a more accurate (and thus more useful) estimated HbA1C value e.g., having less than 3% CV, a more accurate estimated true mean bG is needed. However, it is to be appreciated that in the case of spot measurement devices, increasing the data set of bG measurements upon which to base a more accurate estimated true mean bG is not practical as a PwD can only tolerably comply with about 3 to 6 measurements daily. Additionally, bG values are used in intensive insulin therapy to primarily regulate glucose to target. This means bG measurement timings are dependent on the requirements of intensive therapy and not on providing a better estimate of true mean bG. This is especially true in the case of Type I diabetic patients. Furthermore, for practical considerations the bG measurement cannot be limited to a specific time instant. And finally, the data analyzed normally covers two consecutive patient visits to the HCP. The period between visitations can range from 3 to 4 months. Accordingly, with the above issues in mind, specifying a time window for bG measurement was determined by the inventors to be more realistic. These issues were examined analytically using a gamma function profile, which is discussed hereafter in later sections. An example of a normal lifestyle of a PwD is now provided in order to illustrate the lifestyle aspects and context-based measurements that are collected according to a measurement schema of the present disclosure.

*Lifestyle aspects and Context based measurements*

Intensive therapy addresses the occurrences of bG excursion and provides insulin dosing rules for correcting events such as meals, exercise, medication, etc. This leads to the term "lifestyle" which captures the properties/characteristics of the occurrences of meal events, exercise events, medication events, etc., for a PwD. Lifestyle thus has a strong connotation of daily habits. In the following example, the habits are limited to meals, but other embodiments may be extended to include other events captured, for example, physical activity, intake of oral drugs, and other daily activities.

In the following example, one daily lifestyle pattern (habit) examined consisted of an overnight period and a day period consisting of multiple meals and snacks for a patient. The daily lifestyle pattern repeats itself over a period of months whereby the timing of meals varied randomly around expected meal times. The size and composition of the meal was similarly modeled by assigning the parameters of the gamma function values generated from statistical distribution. In general, it was assumed that by considering more or less a 3 month time frame, the persistent average behavior would be observed in HbAIC value even though from meal to meal there could be potentially large variability. Thus, in the given example, a modal day consisted of an overnight period and 3 meal types: breakfast, lunch and supper as is shown in FIG. 5, which is an example of a normal lifestyle for an individual. It is to be appreciated that snacks are ignored in this illustrated embodiment for simplicity but such can be introduced in other embodiments without impacting the general approach. From either questionnaire or systematic data collection, time periods covering these events are collected.

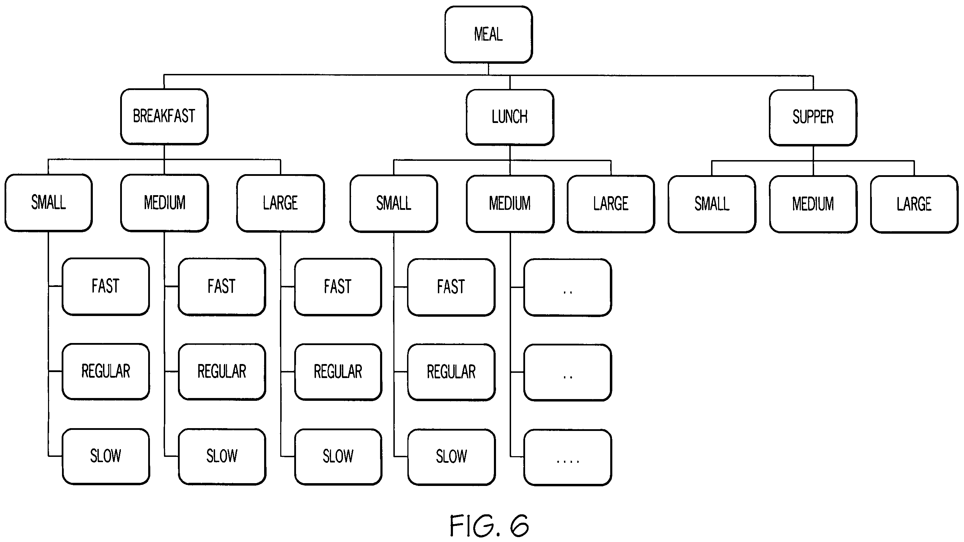

Further complexity in glucose excursion characteristics is addressed by modeling a range of meal content characterized generally by amount and speed. Meal content is given by meal composition and amount of meal which relates to speed and duration of glucose absorption. It is observed that individuals have repeatability in their meal selection, which from a glycemic excursion perspective can be classified by its speed and amount. In one embodiment, as shown in FIG. 6, meals are categorized per meal type, meal amount, and meal speed. Similarly, in other embodiments, additional meals (or less meals if such more accurately reflects a patient's eating habits), exercise (physical activity), stress, alternate states, and medication can be characterized and modeled. For example, an alternate state category may be used to capture the change in physiological metabolic state, such as brought on by stress, a menstrual cycle, exercise, or medication which leads to change in insulin resistance, insulin sensitivity, glucose utilization, and so forth.

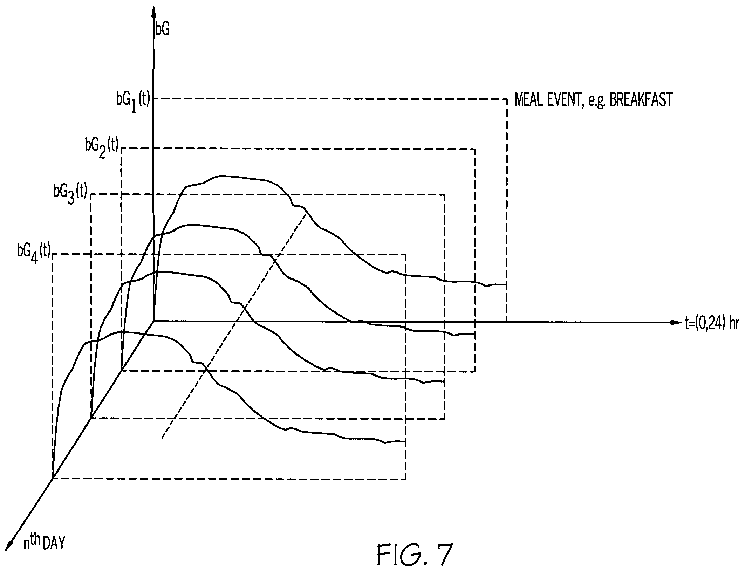

Mathematically, then, statistical properties were assigned to each of the categories. As per the above description, meals were further classified by 3 broad categories of meal speed: fast, regular and slow, and meal amount is similarly classified into 3 categories as: small, medium and large. Other terms used were less than normal, normal and more than normal. While specific categories are presented herein, it should be appreciated that other additional or alternative categories may be used to provide a generalization of a different problem. The latter part described better the majority of the cases. For purposes of simplification of the simulation, physical activity was assumed to be fixed. Thus, grouping glucose sections by event type, for instance meals, and further sub-grouping them by characterizing the meal size and speed, allows one to characterize and quantify them, which is illustrated by FIG. 7. Such context based grouping and then examining the average behavior helped to identify parameters that provided a high correlation ratio. Using the normal lifestyle described above, along with the nine meal categories, a fairly wide range of post prandial behavior for an individual is covered. The gamma function according to Equation 8 was then used in the analytical analysis as well as in simulation to study the impact of lifestyle in the derivation of the relation between HbA1C and bG measurements. It is to be appreciated that how the lifestyle pattern is sectioned and correlated can be varied based upon each patient observed habits and by using other distribution methods in other embodiments.

It is to be appreciated that in reality the glucose profiles of a PwD are richer in their response and are potentially harder to characterize. The richness is associated with multiple physiological factors influencing the overall glucose state. However, assuming that the meal related glucose push is dominant, the PwD is working towards regulating glucose to a target value by means of medications, diet control and exercise and their combinations. Inherently there is an objective of achieving euglycemia at all times. By averaging many such responses, however, the glucose effect on glycemia can be estimated based on the relations derived using the gamma function. The arbitrary meal response curves shown in Fig. 7 are thus represented by the gamma function (Equation 8), which was then used to derive a relation for true mean bG and peak amplitude A. Additionally, the following provides a theoretical basis for the new lifestyle based approach.

*Mean value for Gamma Function*

It is to be appreciated that the gamma function $f(t)$ is neither symmetric nor periodic. We define parameter T which is the time duration between consecutive meal. Considering the exponential properties, the decay of a pure exponential curve to 99% of its starting value is equal to 4 times the time constant. Therefore, the gamma function according to Equation 8 is basically a $2^{nd}$ order differential equation with repeated poles. The time constant for the gamma function is thus , and the mean value can be determined by considering the waning factor n, which is defined as:

$$n = \frac{T}{\beta}$$

or $T = n^*\beta$, where n=3, 4.

The mean bG value for

$$\frac{Ae}{\beta} te^{-t/\beta}$$

(from Equation 8) is now derived. If we consider,

$$g(t) = \frac{Ae}{\beta} \frac{1}{T} \int_0^T te^{-t/\beta} dt,$$

, and integrate $g(t)$ by parts, the following Equations (9)-(13) are provided:

$$g(t) = \frac{Ae}{\beta} \left( \frac{1}{T} \left[ t \frac{e^{-t/\beta}}{-t/\beta} \right]_0^T - \frac{1}{T} \int_0^T (1) \frac{e^{-t/\beta}}{-t/\beta} dt \right), \qquad (9)$$

$$g(t) = \frac{Ae}{\beta} \left( \frac{1}{T} \left[ t \frac{e^{-t/\beta}}{-t/\beta} \right]_0^T - \frac{1}{T} \left[ \frac{e^{-t/\beta}}{(-t/\beta)(-t/\beta)} \right]_0^T \right), \qquad (10)$$

$$g(t) = \frac{Ae}{\beta}\left( \frac{1}{T}\left[ t\frac{e^{-t/\beta}}{-1/\beta}\right]_0^T - \frac{1}{T}\left[ \beta^2 e^{-t/\beta}\right]_0^T\right), \qquad (11)$$

$$g(t) = \frac{Ae}{\beta}\left( -\frac{1}{T}\left[ t\beta e^{-t/\beta} + \beta^2 e^{-t/\beta}\right]_0^T\right), \qquad (12)$$

$$g(t) = \frac{Ae}{\beta}\left( -\frac{1}{n\beta}\left[ n\beta\beta e^{-n\beta/\beta} + \beta^2 e^{-n\beta/\beta}\right] + \frac{1}{n\beta}\left[ 0 + \beta^2 e^{-0/\beta}\right]\right), \quad (13)$$

where $T=n\beta$. Note, however, the mean value is a function of $\beta$, but if $T$ is expressed in terms of $\beta$, then $\beta$ falls out, which further simplifies to

$$g(t) = \frac{Ae}{\beta}\left( \frac{1}{n}\beta\left( 1 - (n+1)e^{-n}\right)\right),$$

, and finally,

$$\overline{bG} = \frac{Ae}{n}\left( 1 - (n+1)e^{-n}\right).$$

In this manner, when the waning factor n equals 3, the mean value $\overline{bG}$ is 0.726A, and when the waning factor n equals 4, the mean value $\overline{bG}$ is 0.617A. Thus, the mean value $\overline{bG}$ is a function of amplitude A and the waning factor $n$. Adding the basal glucose level term $bG_{Const}$, the mean value $\overline{bG}$ can be then defined by Equation (14) as:

$$\overline{bG} = \frac{Ae}{n}\left( 1 - (n+1)e^{-n}\right) + bG_{Const} \qquad (14).$$

Thus, if a peak bG value is measured, then the true mean value $\overline{bG}$ could be estimated since the waning factor n given the lifestyle can be determined by

$$n = \frac{DurationBetweenMeal, T}{TimeToPeak, \beta}.$$

So, for a given gamma function one could simply state that for the mean value, $\overline{bG} = kA + bG_{Const}$. Based on simulation of the Mortensen model, as shown by FIG. 2, it is noted that HbA1C is linearly related to true mean bG. Thus. HbAC1 may be defined by Equation (15) as:

$$HbA1C = K\overline{bG} + \text{constant} \qquad (15).$$

From the above derivation, it is also clear that both meal size and meal duration (associated with speed) influences the degree of glycation. Next, a discussion of the process used to characterize a PwD's lifestyle is provided. Equation (15) is central to derivations presented in latter paragraphs. It is to be appreciated that the relationship between estimated mean bG and the parameters are dependent on context and sampling assumptions. Accordingly the parameters in equation (15) (K, constant) can have potentially different values in other situations. Another example to which the above approach can be applied is the estimation of fructosamine based of the biological measurement blood glucose.

*Lifestyle (meal only)*

As discussed above, the day, as per the lifestyle, is divided into appropriate sections where the bG traces for each day are sectioned and each like sections grouped (e.g., FIG. 7). The bG data covering number of days are grouped into sections as mentioned in the above embodiment comprise: a fasting section, a breakfast section, a lunch section, and a supper section. Starting from continuously sampled data, the mean value $\overline{bG}$ is approximately given by Equation (16) as:

$$\overline{bG} = \frac{\sum_{i=1}^{n} bG_i}{n} \qquad (16).$$

For a meal related section, the gamma function is described by the peak value A with respect to the basal or fasting bG and time to peak, $\beta$ for bG. The parameters are summarized in Table 2.

**Table 2: Meal characteristics**

|  | Fast | Regular | Slow |
|---|---|---|---|
| **Small** | $A_S^{BF}$ , $\beta_{Fast}^{BF}$ | $A_S^{LU}$ , $\beta_{Regular}^{LU}$ | $A_S^{SU}$ , $\beta_{Slow}^{SU}$ |
| **Medium** | $A_M^{BF}$ , $\beta_{Fast}^{BF}$ | $A_M^{LU}$ , $\beta_{Regular}^{LU}$ | $A_M^{SU}$ , $\beta_{Slow}^{SU}$ |
| **Large** | $A_L^{BF}$ , $\beta_{Fast}^{BF}$ | $A_L^{LU}$ , $\beta_{Regular}^{LU}$ | $A_L^{SU}$ , $\beta_{Slow}^{SU}$ |

In terms of analysis then, the meals are then characterized to cover a time period, such as for example, a 2-4 month period between HCP visitations, in the following manner. For breakfast type meals, the total number of breakfasts is represented by the term $m^{BF}$, and the ratio of the number of small breakfast meals, medium breakfast meals, large breakfast meals and no breakfast meals are represented by $\alpha_{SMALL}^{BF}$, $\alpha_{MED}^{BF}$, $\alpha_{LARGE}^{BF}$ and $\alpha_{\phi}^{BF}$ , respectively. Total breakfasts $m^{BF}$ can then be defined according to Equation (17) as:

$$\alpha_{SMALL}^{BF} m^{BF} + \alpha_{MED}^{BF} m^{BF} + \alpha_{LARGE}^{BF} m^{BF} + \alpha_{\phi}^{BF} m^{BF} = m^{BF} \qquad (17).$$

Similarly, meal speeds for fast, regular, and slow meals are represented by the terms: $\lambda_{FAST}^{BF}$, $\lambda_{REG}^{BF}$ , and $\lambda_{SLOW}^{BF}$ , respectively. Therefore, total breakfasts $m^{BF}$ can also be defined according to Equation (18) as:

$$\lambda_{FAST}^{BF} m^{BF} + \lambda_{REG}^{BF} m^{BF} + \lambda_{SLOW}^{BF} m^{BF} + \alpha_{\phi}^{BF} m^{BF} = m^{BF} \qquad (18).$$

It is assumed that on average for each meal amount category there is a breakdown for meal speed with the same ratios. In other words, for example, small breakfast meals $m_{SMALL}^{BF}$ can be defined according to Equation (19) as:

$$\lambda_{FAST}^{BF}\alpha_{SMALL}^{BF}m^{BF} + \lambda_{REG}^{BF}\alpha_{SMALL}^{BF}m^{BF} + \lambda_{SLOW}^{BF}\alpha_{SMALL}^{BF}m^{BF} = m_{SMALL}^{BF} \qquad (19).$$

Equation (16) the $bG_i$ terms on the right hand side are grouped as per FIG 5 to derive a simplified mean glucose relationship using relationship $\overline{bG} = kA + hG_{Const}$ for a Gamma function of amplitude $A$ (derived earlier). The FIG 5 in this example consists of overnight and 3 meal sections breakfast, lunch and supper. The overnight part of the day in this example is generally the sleep period. During this period, the physical activity is minimal. Meal affects are waning out, insulin bolus affects are also petering out. There are other effects such as, for example, the dawn phenomenon caused by growth hormones, which are especially dominant in adolescents. Another example covers medication, wherein the effect of medication on the glucose dynamic response on the drugs respective pharmacokinetics and pharmacodynamics is determined. However, it is anticipated that during the overnight period, the overnight mean blood glucose value, represented by the term $\overline{bG}_{ON}$, is converging to a desired target. Accordingly, $\overline{bG}_{ON}$ is the mean bG obtained by considering all bG values covering a fasting section, and covering all the overnight sections. The mean bG component for the overnight section is then given by Equation (20) as:

$$\overline{bG}_1 = \frac{T_{ON}}{24}\overline{bG}_{ON} \qquad (20),$$

where $T_{ON}$ covers time duration for overnight part as illustrated in Fig 5.

What can constitute fasting bG values requires more specifics. For example, pre-meal bG measurements could be grouped as fasting bG values under certain conditions, overnight bG measurements, early morning bG measurements. Average of such measurements approximately represents the mean bG for the overnight period. Then the component required for $\overline{bG}_{ON}$ is given by Equation (21) as:

$$\overline{bG}_{ON} = \overline{bG}_{Fasting} \qquad (21).$$

Next, given the first predictor term $\overline{bG}_{Fasting}$, which covers the overnight period, the remaining are the meals related excursion with respect to $\overline{bG}_{Fasting}$. So each of the meal which are gamma function thus can be defined according to Equation (22) as:

$$\overline{bG} = KA + \overline{bG}_{FASTING} \qquad (22),$$

where $A$ is the peak disturbance with respect to $\overline{bG}_{Fasting}$.

Determination of "A" for the case when various glucose excursions due to different meals is now explained. As explained earlier and summarized by Fig. 5 and Fig. 6 the excursions are due to the 3 normally eaten meals and then each meal characterized by its size and speed. For illustration purpose, consider the breakfast part first. Next, if consider small breakfast meals and include all meal speeds, then the area under the gamma function according to Equation (23) as:

$$\left(\alpha_{SMALL}^{BF}m^{BF}\right)T^{BF}\overline{bG}_{SM}^{BF} - \overline{bG}_{FASTING} = T^{BF}\sum_{i=1}^{\alpha_{SMALL}^{BF}m^{BF}} K_i^{BF}A_{SMALL,i}^{BF} \qquad (23),$$

which covers all small breakfast meals. The term $T^{BF}$ is the time duration between start of breakfast to start of lunch. Similar equations can be written for medium and large breakfasts, which when combined results in Equation (24), which is defined as:

$$m^{BF}T^{BF}\overline{bG} - \overline{bG}_{FASTING} = T^{BF}\sum_{i=1}^{\alpha_{SMALL}^{BF}m^{BF}} K_i^{BF} A_{SMALL,i}^{BF} + T^{BF}\sum_{i=1}^{\alpha_{MED}^{BF}m^{BF}} K_i^{BF} A_{MED,i}^{BF} + \ldots$$
$$\underbrace{\qquad\qquad}_{Term-1} \qquad \underbrace{\qquad\qquad}_{Term-2}$$

$$T^{BF}\sum_{i=1}^{\alpha_{LARGE}^{BF}m^{BF}} K_i^{BF} A_{LARGE,i}^{BF} + T^{BF}\sum_{i=1}^{\alpha_\phi^{BF}m^{BF}} K^{BF} A_\phi^{BF}$$
$$\underbrace{\qquad\qquad}_{Term-3} \qquad \underbrace{\qquad\qquad}_{Term-4}$$

$$(24).$$

The term $A_\phi^{BF}$ is of course zero. The number of meals considered in the equation covers a time window of interest. Such a window may range from 2 months to 4 months, or may be as few as 7 day to 30 days, if an estimated prediction is desired as explained in a later section.

If Term-1 is considered, then the term $K_i^{BF}$, meal speed, can now be factored out as a constant. The result is shown by Equation (25).

$$\sum_{i=1}^{\alpha_{SMALL}^{BF}m^{BF}} K_i^{BF} A_{SMALL,i}^{BF} = K_{FAST}^{BF}\sum_{i=1}^{\lambda_{FAST}^{BF}\alpha_{SMALL}^{BF}m^{BF}} A_{SMALL,i}^{BF} + K_{REG}^{BF}\sum_{i=1}^{\lambda_{REG}^{BF}\alpha_{SMALL}^{BF}m^{BF}} A_{SMALL,i}^{BF} + K_{SLOW}^{BF}\sum_{i=1}^{\lambda_{SLOW}^{BF}\alpha_{SMALL}^{BF}m^{BF}} A_{SMALL,i}^{BF}$$

$$(25).$$

It is to be appreciated that the PwD categorizes and provides the size of meals as small, medium large meal amounts, as well as the meal speed. For instance, all small meals can be simply represented by an average value $\overline{A}_{SMALL}^{BF}$. Thus, for example, all fast small meals may be represented by Equation (26) as:

$$\lambda_{FAST}^{BF}\alpha_{SMALL}^{BF}m^{BF}\overline{A}_{SMALL}^{BF} \qquad (26).$$

Collecting all the terms together, Equation (25) then can be rewritten as Equation (27) as:

$$\sum_{i=1}^{\alpha_{SMALL}^{BF}m^{BF}} K^{BF} A_{SMALL}^{BF} = \alpha_{SMALL}^{BF}m^{BF}\left(\lambda_{FAST}^{BF}K_{FAST}^{BF} + \lambda_{REG}^{BF}K_{REG}^{BF} + \lambda_{SLOW}^{BF}K_{SLOW}^{BF}\right)\overline{A}_{SMALL}^{BF} \quad (27).$$

Now considering all the meal types we get the following relation shown by Equation (28) is follows:

$$m^{BF}T^{BF}\overline{bG} - \overline{bG}_{FASTING} = \underbrace{T^{BF}\alpha_{SMALL}^{BF}m^{BF}\left(\lambda_{FAST}^{BF}K_{FAST}^{BF} + \lambda_{REG}^{BF}K_{REG}^{BF} + \lambda_{SLOW}^{BF}K_{SLOW}^{BF}\right)\overline{A}_{SMALL}^{BF}}_{Term-1} +$$

$$\underbrace{T^{BF}\alpha_{MED}^{BF}m^{BF}\left(\lambda_{FAST}^{BF}K_{FAST}^{BF} + \lambda_{REG}^{BF}K_{REG}^{BF} + \lambda_{SLOW}^{BF}K_{SLOW}^{BF}\right)\overline{A}_{MED}^{BF}}_{Term-2} +$$

$$\underbrace{T^{BF}\alpha_{LARGE}^{BF}m^{BF}\left(\lambda_{FAST}^{BF}K_{FAST}^{BF} + \lambda_{REG}^{BF}K_{REG}^{BF} + \lambda_{SLOW}^{BF}K_{SLOW}^{BF}\right)\overline{A}_{LARGE}^{BF}}_{Term-3} +$$

$$\underbrace{T^{BF}\sum_{i=1}^{\alpha_\phi^{BF}m^{BF}}K^{BF}A_\phi^{BF}}_{Term-4}$$

(28).

On further simplification, Equation (28) becomes:

$$\overline{bG}^{BF} - \overline{bG}_{FASTING} = \left(\lambda_{FAST}^{BF}K_{FAST}^{BF} + \lambda_{REG}^{BF}K_{REG}^{BF} + \lambda_{SLOW}^{BF}K_{SLOW}^{BF}\right)\left(\alpha_{SMALL}^{BF}\overline{A}_{SMALL}^{BF} + \alpha_{MED}^{BF}\overline{A}_{MED}^{BF} + \alpha_{LARGE}^{BF}\overline{A}_{LARGE}^{BF}\right)$$

The last group of terms on the right-hand side are the weighted amplitude terms which is the average amplitude. Thus, equation (28) can be further rewritten as:

$$\overline{bG}^{BF} - \overline{bG}_{FASTING} = \left(\lambda_{FAST}^{BF}K_{FAST}^{BF} + \lambda_{REG}^{BF}K_{REG}^{BF} + \lambda_{SLOW}^{BF}K_{SLOW}^{BF}\right)\overline{A}^{BF} .$$

And

$$\left(\lambda_{FAST}^{BF}K_{FAST}^{BF} + \lambda_{REG}^{BF}K_{REG}^{BF} + \lambda_{SLOW}^{BF}K_{SLOW}^{BF}\right)$$

is a factor for given lifestyle characteristics. Similarly for other meals, relations can be derived, such as:

$$\overline{bG}^{LU} - \overline{bG}_{FASTING} = \left(\lambda_{FAST}^{LU}K_{FAST}^{LU} + \lambda_{REG}^{LU}K_{REG}^{LU} + \lambda_{SLOW}^{LU}K_{SLOW}^{LU}\right)\overline{A}^{LU} ,$$

and

$$\overline{bG}^{SU} - \overline{bG}_{FASTING} = \left(\lambda_{FAST}^{SU}K_{FAST}^{SU} + \lambda_{REG}^{SU}K_{REG}^{SU} + \lambda_{SLOW}^{SU}K_{SLOW}^{SU}\right)\overline{A}^{SU} .$$

. So the final mean value of bG for a modal day can be defined according to Equation (29) as:

$$\overline{bG} = \frac{T^{FASTING}}{24}\overline{bG}_{FASTING} + \frac{T^{BF}}{24}\overline{bG}^{BF} + \frac{T^{LU}}{24}\overline{bG}^{LU} + \frac{T^{SU}}{24}\overline{bG}^{SU}$$

(29).

The mean values $\overline{bG}^{BF}$, $\overline{bG}^{LU}$ and $\overline{bG}^{SU}$ represent mean bG values for their corresponding meal sections. The above result Equation (29) shows that the specifics of the meal in the final meal equation collapse into a simple average relation in which the averages of an individual event is time weighted as is shown by Equation (29). The above conclusion according to the present disclosure was verified in simulation (FIG. 4). The relation provided by Equation (29) forms the basis to section the day as per lifestyle event and examine it from the perspective of replacing it by a meaningful average value. Alternatively, the mean value of bG for a modal day can be kept in its component parts such that the mean value for each component (*e.g.*, $\overline{bG}_{Fasting}$, $\overline{bG}^{BF}$, $\overline{bG}^{LU}$ and $\overline{bG}^{SU}$) can be utilized to determine the estimated HbA1C value for that particular component using the linear relationship between mean bG and estimated HbA1C as discussed herein. The estimated HbA1C values for each component may then be combined to determine the overall HbA1C for a given day according to Equation (30):

$$HbA1C_{Breakfast} + HbA1C_{Lunch} + HbA1C_{Supper} + HbA1C_{Fasting} = HbA1C \qquad (30)$$

While the above equation breaks down the estimated HbA1C values into time-based components (i.e., breakfast, lunch, supper and fasting) which combine into a complete day, the estimated HbA1C values may alternatively or additionally be broken into other variable-based components (e.g., event-based components or context-based components) as will become appreciated herein. For example, Equation (29) can be rewritten as Equations (29A) and (29B):

$$\overline{bG} = \overline{bG}_{FASTING} + \frac{T^{BF}}{24}\left(\overline{bG}^{BF} - \overline{bG}_{FASTING}\right) + \frac{T^{LU}}{24}\left(\overline{bG}^{LU} - \overline{bG}_{FASTING}\right) +$$
$$\frac{T^{SU}}{24}\left(\overline{bG}^{SU} - \overline{bG}_{FASTING}\right) \qquad (29A).$$

$$\overline{bG} = \overline{bG}_{FASTING} + \frac{T^{BF}}{24}\Delta\overline{bG}^{BF} + \frac{T^{LU}}{24}\Delta\overline{bG}^{LU} + \frac{T^{SU}}{24}\Delta\overline{bG}^{SU} \qquad (29B).$$

Likewise, similar to Equations (29A) and (29B), Equation (30) can be rewritten as Equation (30A) in terms of HbA1C:

$$\Delta HbA1C_{Breakfast} + \Delta HbA1C_{Lunch} + \Delta HbA1C_{Supper} + \Delta HbA1C_{Fasting} = HbA1C \qquad (30A).$$

Another fundamental aspect to the algorithm is temporal weighting schema. The affect of past breakfast on current HbA1C is not equally weighted. Such weight schema is theoretically derived based on the assumption of lifespan of the erythrocytes. As discussed above, this approach can similarly extended to lipid profile, insulin profile, fructosamine and other metabolites

*Temporal Weighting*

Temporal weighting of bG values becomes relevant when the prediction model is derived between SMBG values and HbA1C. As mentioned previously above, each cohort has a finite life span of approximately 120 days. Thus, for this example, a lifespan of 120 days is considered. The aged cells are constantly being replaced by young erythrocytes. So at any given time each of the cohort's age will range from 0 to 119 days. Each cohort thus is exposed to a subset of corresponding bG data. Considering the glycated hemoglobin at current time and all the bG values over the last 120 days, then the bG value that is 120 days old influences only 1 out of 120 cohorts and none of the other cohorts with ages less than 120 days. On the other hand, the current bG value affects all ages of the surviving cohorts i.e. the last 120 cohorts. In context of constant bG for $i^{th}$ day and considering the physiological aspect, this suggests that an appropriate weighted mean bG value can help improve HbA1C prediction.

In a simulation exercise, a lifespan L was set to 120 days and a number of cohorts N was made equal to 120 cohorts, where cohort # 120 is the oldest cohort, and cohort # 1 is the newest. For a cohort aged L days (considering the oldest cohort), then the impact of $bG_i$ on HbA1C can be approximated according to Equation (31) as:

$$\frac{\sum_{i=1}^{L} bG_i \Delta T}{\sum_{i=1}^{L} \Delta T} = \frac{1}{L}\sum_{i=1}^{L} bG_i \qquad (31),$$

where AG, is glucose value on $i^{th}$ day, where index i is 1, 2, 3, ...L, from the latest glucose measurement to oldest glucose measurement. Similarly, for a cohort aged L-1 day, mean bG can be defined according to Equation (32) as:

$$\frac{\sum_{i=1}^{L-1} bG_i \Delta T}{\sum_{i=1}^{L-1} \Delta T} = \frac{1}{L-1}\sum_{i=1}^{L-1} bG_i \qquad (32).$$

And so on. Collecting weights for same $bG_i$, the weights may be defined according to Equation (33) as:

$$\left[ \left(\frac{1}{L}\right)bG_L \quad \left(\frac{1}{L}+\frac{1}{L-1}\right)bG_{L-1} \quad ... \quad \left(\frac{1}{L}+\frac{1}{L-1}+...+\frac{1}{1}\right)bG_1 \right] \qquad (33).$$

It is to be appreciated that the above weighting scheme corresponds to a harmonic series. As such, the weights will be referred to herein as harmonic weighting.

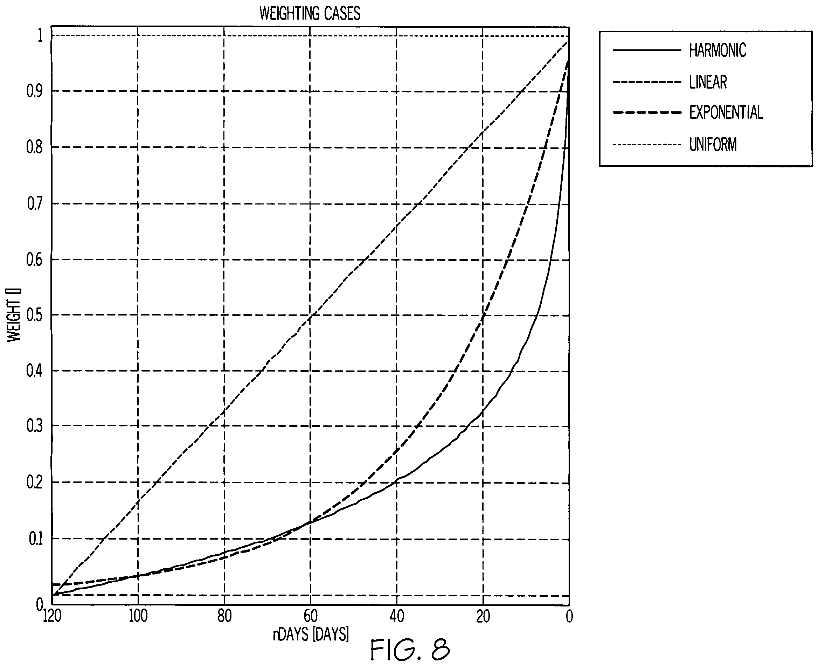

FIG. 8 shows 4 weighting schemes that were considered in developing the prediction model for HbA1C. From using the harmonic weighting in the above Equation (33), it is clear that older bG values contribute progressively less and less to HbA1C value. If the area under the harmonic curve is considered, then the period covering 60 days represents 84.4% of the total, which is shown in Table 3.

**Table 3: Area under the harmonic curve**

| Visitation Period | Percentage Area |
|---|---|
| From=1 To=1 | 0 |
| From=10 To=1 | 28.1 |
| From=20 To=1 | 45.7 |
| From=30 To=1 | 59.0 |
| From=40 To=1 | 69.5 |
| From=50 To=1 | 77.8 |
| From=60 To=1 | 84.4 |
| From=70 To=1 | 89.6 |
| From=80 To=1 | 93.6 |
| From=90 To=1 | 96.5 |
| From=100 To=1 | 98.5 |
| From=110 To=1 | 99.6 |
| From=120 To=1 | 100 |

Additional results showed that harmonic temporal weighting is a relevant scheme in the determination of the HbA1C estimate based on SMBG measurements, and that the period over which SMBG data contributes significantly to estimating HbA1C is about 60 days (considering in this case life of erythrocytes as 120 days. Similar reasoning can be used when considering erythrocytes for other ages). Analysis results also supports that collecting bG values collected over a visitation period of about approximately 60 days provides the best estimate on HbA1C. In one embodiment, the collecting of both bG measurements and associated context of the bG measurement at daily times specified by the structured sampling schema is over a period of about 2 to about 4 months. In another embodiment, a small time window such as ranging from 1 week to 4 weeks can be used as a representative of glucose behavior covering a 3 to 4-month period. This allows the HCP and patient to revise the current therapy or behavior to try achieving prescribed targeted goals. The resulting predicted HbA1C then represents a future HbA1C which provides the patient and/or HCP the future glycemic level is assuming the current glucose behavior is maintained. The principles behind the process used to derive a correlation coefficient for meal sections according to the present disclosure is now discussed hereafter.

*Correlation coefficient for meal sections*

Glucose data collected during two independent clinical studies in 2003 and 2006 were used to determine a correlation coefficient for meal sections from which to devise a sampling schema for use with the HbA1C prediction model. The clinical trials studied the post prandial glucose control for meals with different meal composition. The key aspects of each of the two studies are summarized below.

*Meal study 2003*

1. Study was conducted during 2003-2004. The study was designed to examine the meal response of fixed insulin bolus to meals with varying glucose absorption characteristics.

2. Demographics of the subjects participating in the study are:

   a. Number of Subjects=23
   b. Number of study blocks=4
   c. Number of males=12, Number of females=11
   d. Age (40$\pm$9) years
   e. Weight (75$\pm$15) kg
   f. BMI (24.6$\pm$2.5) kg/m$^2$
   g. HbA1C (7.0$\pm$1.0)%

3. Each visit is 4 days long:

   a. Day 1:

      i. Subject arrives in the evening to be instrumented.
      ii. Has an evening supper
      iii. Spot monitoring

   b. Day 2:

      i. 9 am Test meal (A, B, C, D, E and F)
      ii. 3 pm late lunch meal
      iii. 7 pm supper

   c. Day 3:

      i. 9 am Test meal (A, B, C, D, E and F)
      ii. 3 pm late lunch meal
      iii. 7 pm supper

   d. Day 4:

      i. Subject leaves around breakfast time

4. Number of study blocks is 4. A study block is the re-visitation of the subject for performing the meal study with a different test meal and/or insulin therapy algorithm.

5. Meal sections were extracted from Meal Study 2003. The sections were of duration:

    a. 6 hr, all test meals (@ 9:00 am)
    b. 4 hr, all late lunch (@ 3:30 pm)
    c. 8 hr, all supper (@ 7:00 pm)

*Meal study 2006*

1. Study was conducted during the year 2006-2007
2. Demographics of the subjects:

    a. Number of Subjects= 12
    b. Number of study blocks=4
    c. Number of males=7, Number of females=5
    d. Age (45±9) years
    e. Weight (75±14) kg
    f. BMI (24.7±3.0) kg/m$^2$
    g. HbA1C (6.9 ±0.8)%

3. Each visit (block) is 4 days long:

    a. Day 1:

        i. Subject arrives in the evening to be instrumented.
        ii. Has a evening supper
        iii. Spot monitoring

    b. Day 2:

        i. 9 am Test meal (A, B, E and F)
        ii. 3 pm late lunch meal
        iii. 7 pm supper

    c. Day 3:

        i. 9 am Test meal (A, B, E and F)
        ii. 3 pm late lunch meal
        iii. 7 pm supper

    d. Day 4:

        i. Subject leaves around breakfast time

4. Number of study blocks is 4. A study block is the re-visitation of the subject for performing the meal study with a different test meal and/or insulin therapy algorithm.
5. Meal sections were extracted from Meal Study 2006. The sections were of duration:

    a. 6 hr, all test meals (@ 9:00 am)
    b. 4 hr, all late lunch (@ 3:30 pm)
    c. 8 hr, all supper (@ 7:00 pm)

The above test meal labels A-F describe the meal speed. Meals labeled A and B are fast meals, meals labeled C and D are regular, and meals labeled E and F are slowly absorbing meals. The meals were classified by a professional dietician. The meal study data set provided discrete frequently measured bG data, where the sampling rates for the time window covering the test meals were 10 minutes. Sampling rates at other times range from 1 minute to as rarely as hourly measurement, such as overnight. Also available in the bG data is specific insulin, ingested mixed meal information and interventions. It is to be appreciated that the clinical bG data set did not include HbA1C values. HbA1C values were then generated artificially by using the Mortensen model (Equations (1)-(3)) with the bG data.

It is clear from earlier analysis that there exists a linear relationship between true mean bG and HbA1C. It is then clear that one could simply focus on the question of determining either true mean bG or HbA1C. Given the continuous and/or frequent bG measurements in the bG data, the bG curves were then sectioned into relevant groups and correlation between various parameters such as minimum, maximum, glucose value at specified time and so forth were correlated to true mean bG as well as HbA1C.

In regards to HbA1C, this value was determined by inputting the glucose curve to the Mortensen model Equations (1)-(3). In this regard then, the meal data was first divided into meal sections. Each of the meal sections was curve fitted and then the resulting signal was repeated to create as input a bG input signal of duration 150 days. The resulting profile was then passed through the Mortensen model. (Equations (1)-(3)) to generate the HbA1C values. In this way, HbA1C for each of the meal sections was generated.

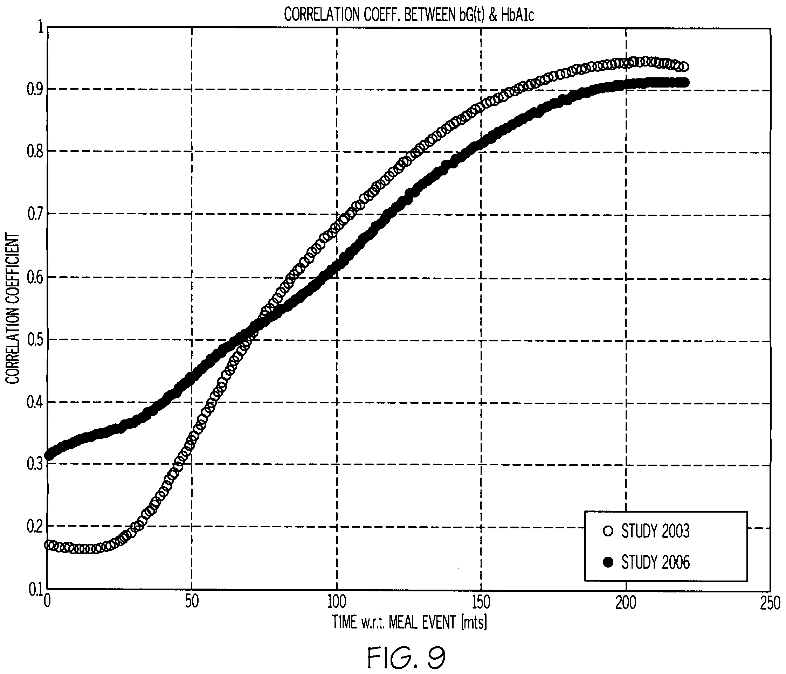

Next, several predictors were examined to correlate with HbA1C. The most meaningful single-point predictor discovered by the inventors was a bG measurement taken at a particular post-prandial time point. For this predictor, a Pearson correlation coefficient was used as a function of bG(t) which is shown plotted in FIG. 9. The correlation coefficient for meal sections extracted from the clinical meal studies of 2003 and 2006 shows that there is a strong linear relationship between HbA1C and post prandial bG measurement when t≥150 minutes.

Although the correlation coefficients may differ for different clinical studies, in general the trends are expected to be similar. As shown by FIG. 9, a low correlation is seen in the 1st hour; the correlation then starts increasing and reaches values greater than 0.8 for 2.5 hrs postprandial. Such variation could be explained by meal type, meal amount and associated insulin therapy. Low correlation in early hours of postprandial is due to transients caused by variations due to meal glucose absorption and due to insulin absorption characteristics. As the transients die out the correlation increases. The increased correlation for the clinical studies is also due to following reasons: the subjects are well motivated, so in general, their glycemic excursion should recover quite consistently for subjects during postprandial period.

The variations in meal behavior are due to main factors such as physiology, meal content variation, inaccuracies in physiological parameter estimates, basal setting. The correlation coefficients indicate that meals correlate to HbA1C very strongly when bG measurements are conducted postprandially in time range around 3 hours. It is also clear from simulation that the transient bG has comparatively less impact than the steady state behavior of the meal that is the relatively slow and steady push. The variability in the early transients is clearly indicative of lack of specific knowledge of day to day physiological variability and imprecise knowledge of meal but the general control strategy on the latter post prandial state is important in achieving low HbA1C. While this method was described with respect to HbA1C, the general method also provides in detail how one can extend the approach to cover other biological values (e.g., metabolites and biomarkers) such as fructosamine (a biomarker for glycemia over past 3-4 weeks, where fructosamine is glycated albumin). Furthermore, solutions for problems under different assumptions can be redone to derive estimation relations and/or the parameters.

The following section hereafter focuses on deriving an optimal sampling schema for determination of true mean bG and HbA1C. Sampling schema is determined by using the equations developed in earlier sections, such as lifestyle related time weighting addressing a modal day, and glucose weighting addressing the data covering a visitation period (i.e., period between visitations).

*Structured Sampling Schema*

Using clinical data from Meal study 2003, bG profiles are generated by combining various meal sections by randomly selecting bG profile sections from different meal bins and concatenating the sections. The various meal bins are listed in Table 4.

**Table 4: Meal Bins**

|  | **Breakfast** | **Lunch** | **Supper + Overnight** |
|---|---|---|---|
| **Low - HbA1C** | First 1/3rd of ranked Breakfast meals | First 1/3rd of ranked lunch meals | First 1/3rd of ranked supper meals |
| **Medium - HbA1C** | Second 1/3rd of ranked Breakfast meals | Second 1/3rd of ranked lunch meals | Second 1/3rd of ranked supper meals |

(continued)

|  | Breakfast | Lunch | Supper + Overnight |
|---|---|---|---|
| High - HbA1C | Third 1/3rd of ranked Breakfast meals | Third 1/3rd of ranked lunch meals | Third 1/3rd of ranked supper meals |

The bins in Table 4 represent meal sections and are first of all grouped by collecting the sections obtained from breakfast, lunch and supper and overnight time periods. The meal sections were further ranked and sorted in ascending order in terms of corresponding HbA1C values from simulation. The breakfast meal pool was then divided into 3 equal groups by selecting the first one-third breakfast meals and labeled as low - HbA1C, then the second one third of breakfast meals labeled as Medium - HbA1C and the remaining breakfast meals as High - HbA1C. In a similar fashion, lunch and supper are also binned. In all, 9 meal bins were created. To create lifestyle based bG sequence, lifestyle is described as the modal day consisting of breakfast starting at 8 am with one of the HbA1C group (Low, Medium or High); lunch at noon with one of the HbA1C group (Low, Medium or High) and supper at 6 pm with one of the HbA1C group (Low, Medium or High). In this manner, 174 bG sequences were generated covering various combinations.

As mentioned in the previous section, if bG measurements are conducted postprandially around the time interval when the correlation coefficient is high (e.g., $t \geq 150$ minutes, FIG. 9) a good estimate of HbA1C can be anticipated. Therefore, the key factors relating to a useable prediction of HbA1C from a series of bG measurements are the following: (a) timing of bG measurement, (b) number of bG measurements (in range of 2-6 measurements per day), (c) accuracy of predicted A1C, and (d) bias of the predicted A1C.

As per lifestyle (primarily done for meal in the illustrated embodiment) the sampling schema was setup according to Table 5 as follows:

**Table 5: Sampling Schema setup**

| Center of the Sampling window (WinCen) | Determine the optimal expected time at which one should sample for SMBG. |
|---|---|
| Size of the sampling window | The allowance/tolerance to measurement |
| (WinSize) | time window around WinCen. |
| Number of Days (nDays) | SMBG data is collected over period of last nDays days. |
| Number of samples (nSamples) | The bG sampling is event driven. With respect to each meal event, the number of samples collected during the specified number of days, nDays. As an example nSamples=50 means that as described by Lifestyle (Fig. 5) for breakfast there are 50 bG measurements spanning over nDays, then 50 measurements for lunch spanning the nDays and then for supper 50 measurements spanning the nDays. |
| Sampling Ratio, $$\dfrac{nSamples}{nDays}$$ | It is the ratio of number of glucose measurements to the number of days (nDays) over which the samples are collected, for each event type. For example, nSamples=50 and nDays=70 then Sampling ratio=50/70. |

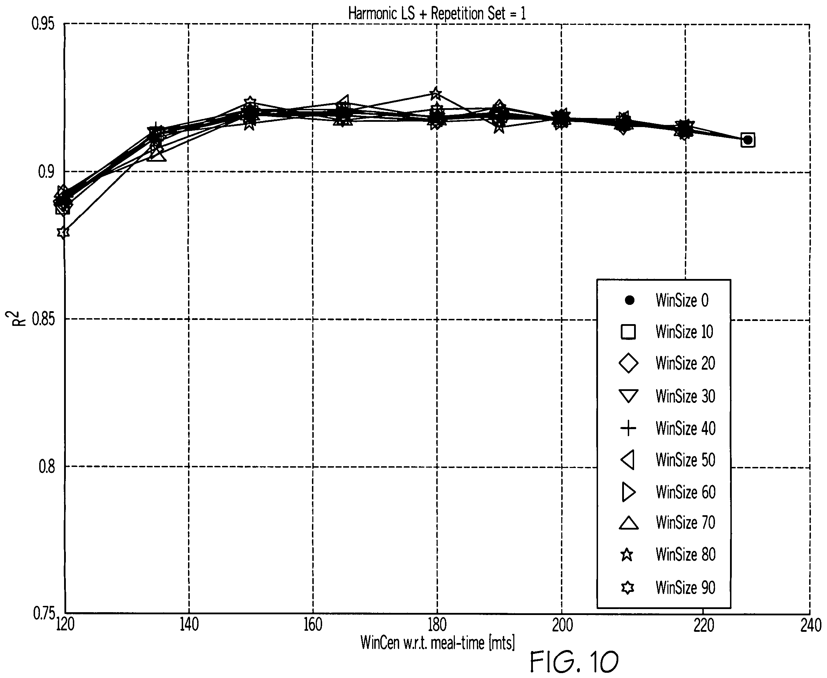

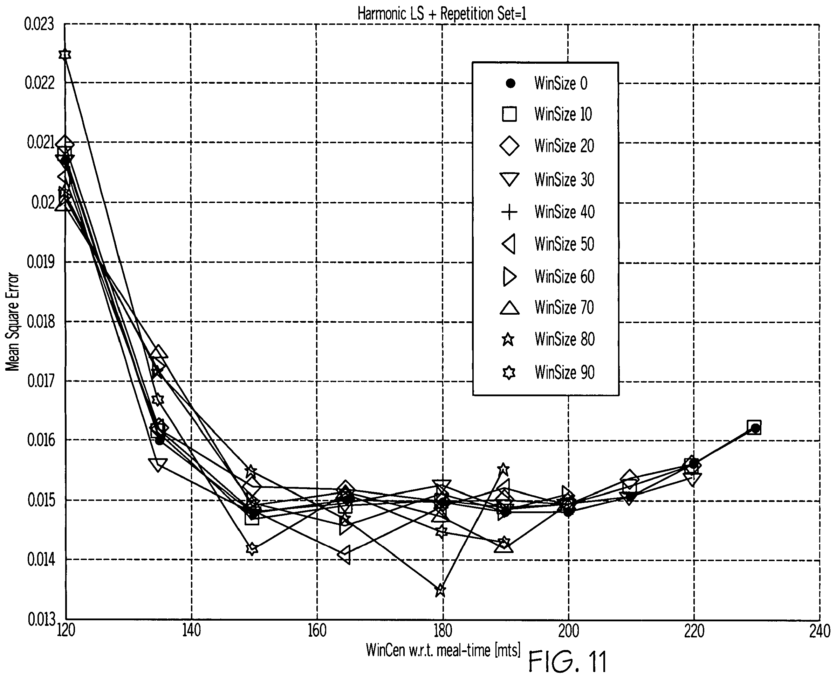

Linear regression was then carried out to predict HbA1C from SMBG measurements, whereby SMBG values were processed by various lifestyle weighting and averaging strategies. FIG. 10 shows the $R^2$ (R-squared value) from the linear regression against HbA1C for various values of WinCen and WinSize for the lifestyle. For the illustrated plot of FIG. 10, the visitation period (nDays) equals 60 days, and the number of samples (nSamples) also equals 60. As shown, the best $R^2$ is centered on post-prandial time of 190 minutes. Similarly plotting the results for mean squared error (FIG. 11) it is observed that a postprandial measurement around 180 minutes provides minimum error in HbA1C estimate.

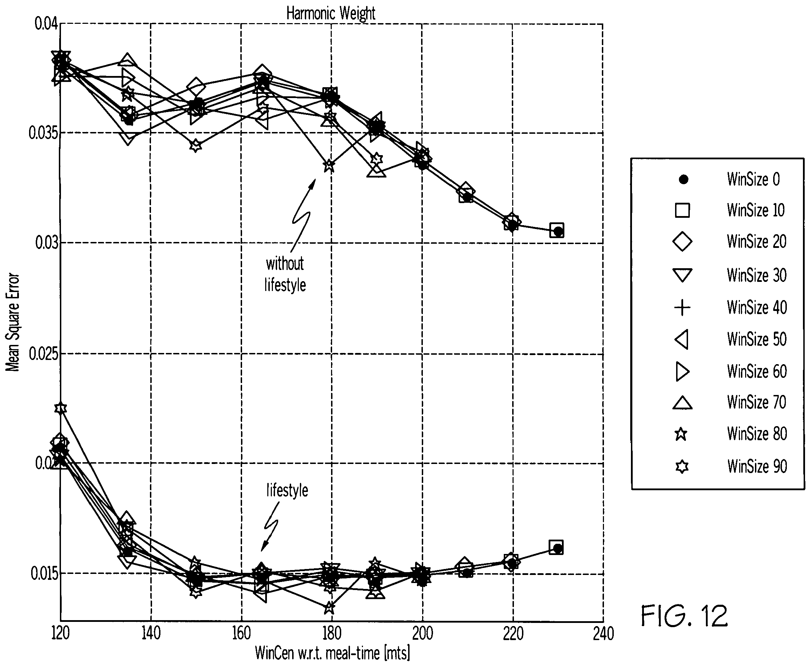

In FIG. 12, a comparison between the daily lifestyle weighting and no daily lifestyle weighting shows that daily life style weighting produces lower MSE (mean squared error).

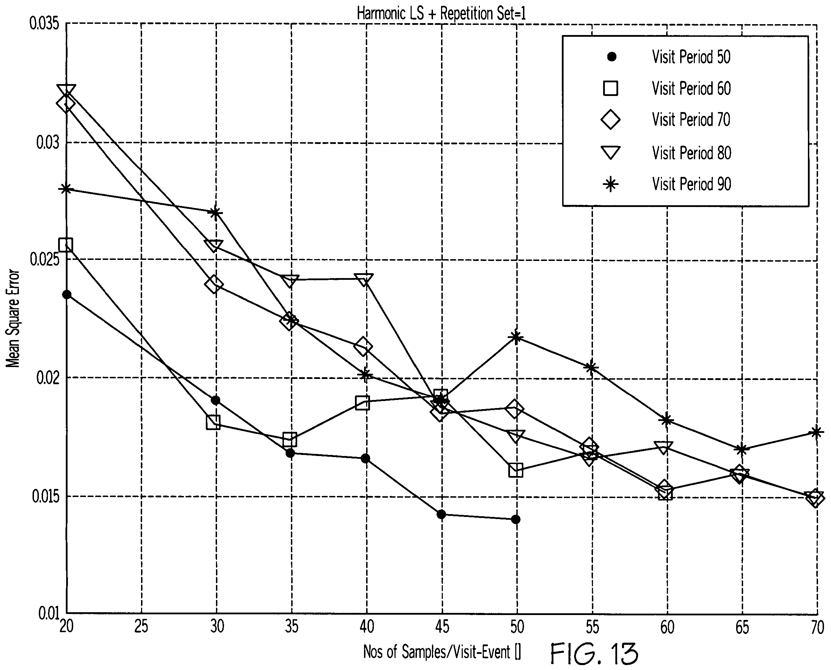

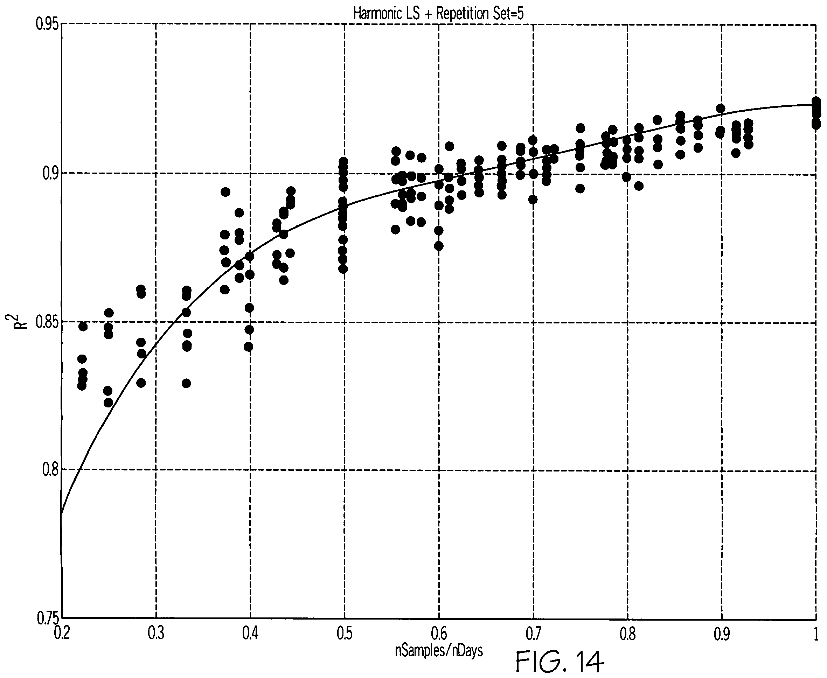

FIG. 13 shows the impact of nDays (visitation period) and nSamples for WinCen of 190 minutes and WinSize of 50 minutes. It shows that MSE reduces as the number of SMBG measurements is increased. In particular, there is an impact of nDays. The number of days shows that there are an optimal number of days beyond which the $R^2$ does not improve. To determine the best nSamples and nDays one actually needed to look at the sampling ratio which is the number of samples per event (nSamples/nDays). The requirement was to have the ratio as small as possible with some acceptable $R^2$. What was observed was that below 0.5 both $R^2$ deteriorate at a rapid pace and also the spread in their values became greater. For a sampling ratio greater than 0.5 and above, the $R^2$ value was > 0.85. For $R^2 > 0.9$, a sampling

ratio $\dfrac{nSamples}{nDays}$ of 0.55 was obtained as shown by FIG. 14.

*Regression Model for estimating HbA1C*

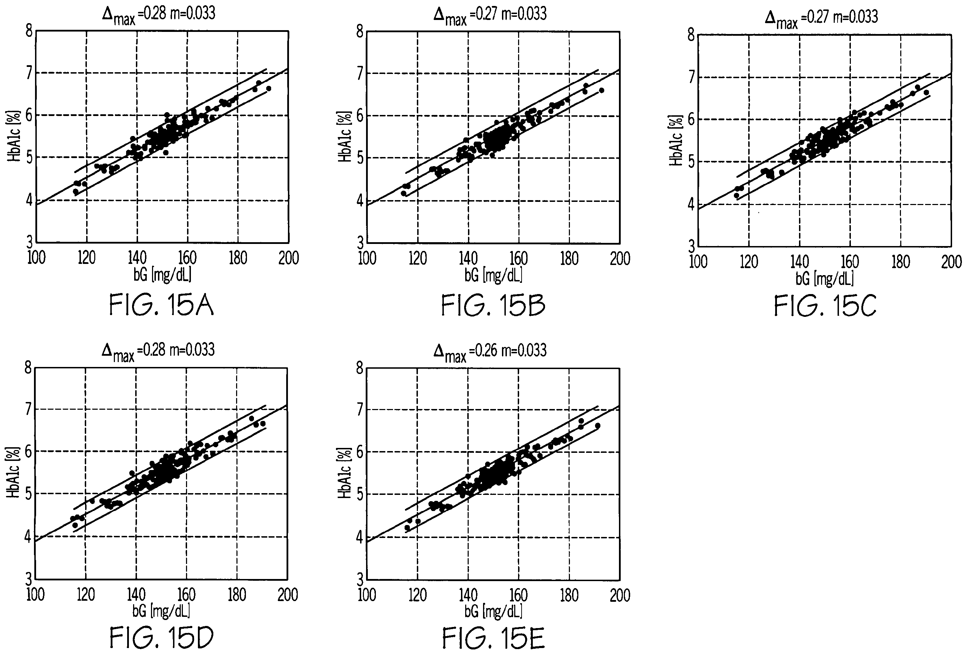

As per the sampling schema, the sampled bG data were then regressed and plotted, which are shown by FIGS. 15A-E. Tabulated results of the regression are provided in Table 6, which shows that the parameters for each linear regression are in close proximity to each other. In FIGS. 15A-E, the prediction line (centerline) and a 95% confidence interval (CI) boundaries (above and below curves) are shown in the subplots. The 95% CI covers a range which deviates approximately 0.26% HbA IC from the nominal value.

**Table 6: Linear regression parameters**

| Figure | Delta | Slope | Intercept |
|---|---|---|---|
| 15A | 0.28 | 0.033 | 0.587 |
| 15B | 0.27 | 0.033 | 0.581 |
| 15C | 0.27 | 0.033 | 0.588 |
| 15D | 0.28 | 0.033 | 0.547 |
| 15E | 0.26 | 0.033 | 0.548 |

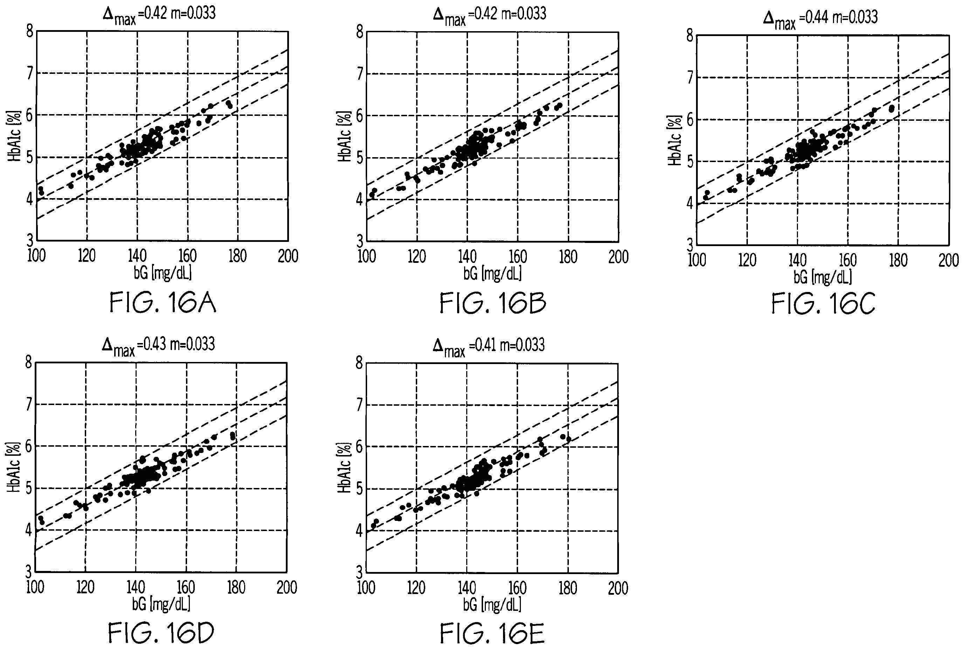

FIGS. 16A-E show that CI boundaries contain almost all of the HbA1C observations. A slope of 0.033 or 1/30 is obtained from the linear regression. In summary, the optimal SMBG sampling parameters along with regression parameters for determining an estimated true mean bG value and estimated HbA1C value is listed in Table 7.

**Table 7: Structured Sampling Schema**

| Parameter | Optimal value |
|---|---|
| WinCen | 190 min |
| WinSize | 50 min |
| nSamples | 45 samples per event |
| nDays | 80 days |
| Weighting function | Harmonic |
| Lifestyle weighting | Yes |
| Estimated HbA1C | $0.033\overline{bG} + 0.5702$ |

The Estimated HbA1c in Table 7 comprises virtual HbA1c determined based of the Mortenson model in which patient specific relationships and estimates can be addressed by calibration as discussed later herein. Alternatively, the weighted component based bG estimates presented herein can be used in HbA1c estimate relations such as the Nathan's relation (Nathan, D. M.; Schoenfeld, D.; Kuenen, J.; Heine, R., J.; Borg, R.; Zheng, H.; "Translating the A1C Assay into estimated average glucose values," Diabetes Care, Vol 31, Nos 8, Aug 2008, pp. 1-6.), which estimate patient HbAlc, or Abensour's relation (U.S. Patent Publication No. US 2007/0010950 A1).

*Validation*

To validate the results obtained above in Table 7, which were derived using the meal sections extracted from the Meal Study 2003, the Meal Study 2006 was then used. Similar to Meal Study 2003, all the meal sections from Meal Study 2006 were extracted. Overall, 286 meal sections were obtained from the 2006 study. All the meal sections were then fitted by a polynomial curve, and ordered in an ascending order by their individual HbA1C values (obtained by using the Mortensen Model). The meal sections were then binned into groupings done in the manner explained for Meal Study 2003. Using the meal sections, a bG sequence covering a duration of 300 days was generated as per the previous lifestyle used in the 2003 meal study. The simulation duration was also set to 300 days. The bG profiles and HbAIC were then stored for sampling and HbA1C prediction. In all 108 simulations were generated.

The bG values were then sampled as per the Sampling Schema listed in Table 7. Using the sampled bG values for each of the 108 simulation cases, mean bG was determined. The relation between the mean bG and HbA1C, as determined by simulation, is plotted in FIGS. 16A-E. Each of the FIGS. 16A-E is a subplot which simply repeats a random sampling as per the schema explained earlier. Each subplot shows the estimated mean bG with the true HbA1C. The upper and lower lines in each subplot indicated 3 standard deviations (SD line) from the predicted HbAC1 algorithm, $0.033\overline{bG}$ + 0.5702, (e.g., center line in each subplot) as determined previously above. It is to be appreciated that the distance between the SD line and the mean behavior on an average is 0.44 % HbA1C. Therefore, as expected there was a degradation (spread) in the estimated HbA1C value, however the resulting precision was still within 3% CV.

From the above results, if a slightly lower $R^2$ of 0.85 is used, then the number of measurements/event can be reduced to 45 over 80 days. With 3 meal events per day plus a nighttime measurement, then the number of measurements equal 180 measurements. This implies approximately 2.25 measurements/day are needed as per sampling schema described above to achieve an estimated HbA1C value that has a precision within 3% CV.

It should be appreciated that the stated derived results are one of many ways of using the approach. The estimated value could alternatively or additionally be plugged into pre-defined models, for instance Nathan's relation as described in Nathan, D. M.; Schoenfeld, D.; Kuenen, J.; Heine, R., J.; Borg, R.; Zheng, H.; "Translating the A1C Assay into estimated average glucose values," Diabetes Care, Vol 31, Nos 8, Aug 2008, pp. 1-6., where the mean bG value based of the analysis presented herein is used in the other model. Nathan's relation, for example, can provide an estimate through a relationship that that is accepted in the medical community while using a better estimate of the mean bG (which should ideally improve the estimate as well as provide better acceptance of the result by the medical community).

*Implementation examples*

The above described sampling schema and prediction algorithm for providing both an estimated true mean blood glucose value and an estimated glycated hemoglobin (HbA1C) value from structured spot measurements of blood glucose may be implemented using hardware, software or a combination thereof. For example, the above described sampling schema and prediction algorithm may be implemented in one or more microprocessor based systems, such as a portable computer or other processing systems, such as personal digital assistants (PDAs), or directly in self-monitoring glucose devices or meters (bG meters) equipped with adequate memory and processing capabilities to process a chronological sequence of measurements of a time dependent parameter measured in or on the human body, namely of the glucose level (e.g. the glucose (bG) level). In some embodiments, remote servers may process the measurements to determine the estimated and/or predicted values and provide these determined values to a personal glucose meter, PDA or the like. In these embodiments, the personal glucose meter, PDA or the like may thereby be operable with a relatively smaller processor that could not as quickly determine the values compared to an application running on the remote server.

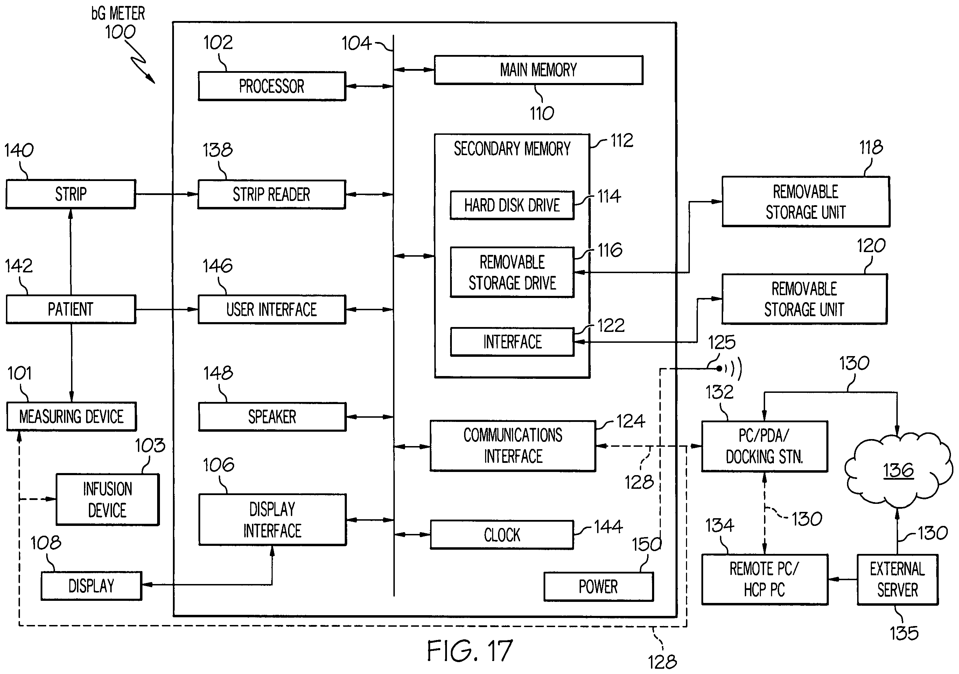

In an example embodiment, the sampling schema and prediction algorithm are implemented in software running on a self-monitoring blood glucose (bG) meter 100 as illustrated in FIG. 17. The bG meter 100 is common in the industry and includes essentially any device that can function as a glucose acquisition mechanism. The bG meter 100 or acquisition mechanism, device, tool, or system includes various conventional methods directed toward drawing a sample (e.g. by finger prick) for each test, and making a spot determination of the glucose level using an instrument that reads glucose concentrations by optical, electrochemical, electromechanical or calorimetric detection/measurement methods. In addition, the bG meter 100 may include and/or communicate with measuring devices 101 capable of measuring one or more biological measurement (e.g., glucose, lipids and/or triglycerides. For example, measuring devices the bG meter 100 can include and/or communicate with devices with indwelling catheters and subcutaneous tissue fluid sampling devices (e.g., a continuous glucose monitor (CGM) device) and/or a drug pump/infusion device 103.

In the illustrated embodiment, the bG meter 100 includes one or more microprocessors, such as processor 102, which is connected to a communication bus 104, which may include data, memory, and/or address buses. The bG meter 100 may include a display interface 106 providing graphics, text, and other data from the bus 104 (or from a frame buffer not shown) for display on a display 108. The display interface 106 may be a display driver of an integrated graphics solution that utilizes a portion of main memory 110 of the bG meter 100, such as random access memory (RAM) and processing from the processor 102 or may be a dedicated graphics card. In another embodiment, the display interface 106 and display 108 additionally provide a touch screen interface for providing data to the bG meter 100 in a well-known manner.

Main memory 110 in one embodiment is random access memory (RAM), and in other embodiments may include other memory such as a ROM, PROM, EPROM or EEPROM, and combinations thereof. In one embodiment, the bG meter 100 includes secondary memory 112 which may include, for example, a hard disk drive 114 and/or a removable storage drive 116, representing a floppy disk drive, a magnetic tape drive, an optical disk drive, a flash memory, etc. The removable storage drive 116 reads from and/or writes to a removable storage unit 118 in a well-known manner. Removable storage unit 118, represents a floppy disk, magnetic tape, optical disk, flash drive, etc. which is read by and written to by the removable storage drive 116. As will be appreciated, the removable storage unit 118 includes a computer usable storage medium having stored therein computer software and/or data.

In alternative embodiments, secondary memory 112 may include other means for allowing computer programs or other instructions to be loaded into the bG meter 100. Such means may include, for example, a other removable storage unit 120 and an interface 122. Examples of such removable storage units/interfaces include a program cartridge and cartridge interface, a removable memory chip (such as a ROM, PROM, EPROM or EEPROM) and associated socket, and other removable storage units 120 and interfaces 122 which allow software and data to be transferred from the other removable storage unit 120 to the bG meter 100.

The bG meter 100 in one embodiment includes a communications interface 124. The communications interface 124 allows software and data to be transferred between the bG meter 100 and an external device(s) 132. Examples of communications interface 124 may include one or more of a modem, a network interface (such as an Ethernet card), a communications port (e.g., USB, firewire, serial or parallel, etc.), a PCMCIA slot and card, a wireless transceiver, and combinations thereof. In one embodiment, the external device 132 is a personal computer (PC), and in another embodiment is a personal digital assistance (PDA). In still another embodiment, the external device 132 is a docking station wherein the communication interface 124 is a docket station interface. In such an embodiment, the docking station may be provided and/or connect to one or more of a modem, a network interface (such as an Ethernet card), a communications port (e.g., USB, firewire, serial or parallel, etc.), a PCMCIA slot and card, a wireless transceiver, and combinations thereof. Software and data transferred via communications interface 124 are in the form of wired or wireless signals 128 which may be electronic, electromagnetic, optical, or other signals capable of being sent and received by communications interface 124. For example, as is known, signals 128 may be sent between communication interface 124 and the external device(s) 132 using wire or cable, fiber optics, a phone line, a cellular phone link, an RF link, an infrared link, other communications channels, and combinations thereof. In some embodiments, the bG meter 100 comprises remote server connection 125 to send data to an external server such that the external server processes the requested information and sends the results back to the bG meter 100 as discussed herein.

In one embodiment, the external device 132 is used for establishing a communication link 130 between the bG meter 100 and still further electronic devices such as a remote Personal Computer (PC) of the patient, and/or a health care provider (HCP) computer 134, or an external server 135 directly or indirectly, such as through a communication network 136, such as the Internet and/or other communication networks. The communication interface 124 and/or external device(s) 132 may also be used to communicate with further data gathering and/or storage devices such as insulin delivering devices, cellular phones, personal digital assistants (PDA), etc. Specific techniques for connecting electronic devices through wired and/or wireless connections (e.g. USB and Bluetooth, respectively) are well known in the art.

In the illustrative embodiment, the bG meter 100 provides a strip reader 138 for receiving a glucose test strip 140. The test strip 140 is for receiving a sample from a patient 142, which is read by the strip reader 138. Data, representing the information provided by the test strip, is provided by the strip reader 138 to the processor 102 which executes a computer program, e.g., provided in main memory 110, to perform various calculations as discussed in great detail below on the data. The results of the processor 102 from using the data is displayed on the display 108 and/or recorded in secondary memory 112 by the processor 102, which is herein referred to as self-monitored glucose (bG) data. The bG data may include, but not limited thereto, the glucose values of the patient 142, the insulin dose values, the insulin types, and the parameter values used by processor 102 to calculate future glucose values, supplemental insulin doses, and carbohydrate supplements. Each glucose value and insulin dose value is stored in memory 112 by the processor 102 with a corresponding date and time. An included clock 144 of the bG meter 100 supplies the current date and time to processor 102. The bG meter 100 further provides a user input device(s) 146 such as keys, touchpad, touch screen, etc. for data entry, program control, information requests, and the likes. A speaker 148 is also connected to processor 102, and operates under the control of processor 102 to emit audible and/or visual alerts/reminders to the patient of daily times for bG measurements and events, such as for example, to take a meal, of possible future hypoglycemia, and the likes. A suitable power supply 150 is also provided to power the bG meter 100 as is well known to make the meter portable.

The terms "computer program medium" and "computer usable medium" are used to generally refer to media such as removable storage drive 116, a hard disk installed in hard disk drive 114, signals 128, etc. These computer program products are means for providing software to bG meter 100. Embodiments of this disclosure include such computer program products.

Computer programs (also called computer control logic) are stored in main memory 110 and/or secondary memory 112. Computer programs may also be received via the communications interface 124. Such computer programs, when executed, enable the bG meter 100 to perform the features of the present disclosure as discussed herein. In particular, the computer programs, when executed, enable processor 102 to perform the functions of the present disclosure. Accordingly, such computer programs represent controllers of bG meter 100. Alternatively or additionally, the computer programs may be stored and/or run on remote servers with input and output data communicated via wired or wireless communication networks.

In an embodiment where the disclosure is implemented using software, the software may be stored in a computer program product and loaded into bG meter 100 using removable storage drive 116, other removable storage unit 120, hard disk drive 114, or communications interface 124. The control logic (software), when executed by the processor 102, causes the processor 102 to perform the functions of the disclosure as described herein.

In another embodiment, the disclosure is implemented primarily in hardware using, for example, hardware components such as application specific integrated circuits (ASICs). Implementation of the hardware state machine to perform the functions described herein will be apparent to persons skilled in the relevant art(s).

In yet another embodiment, the disclosure is implemented using a combination of both hardware and software.

In an example software embodiment of the disclosure, the methods described hereafter are implemented in the C++ programming language, but could be implemented in other programs such as, but not limited to, Visual Basic, C, C#, Java or other programs available to those skilled in the art (or alternatively using script language or other proprietary interpretable language used in conjunction with an interpreter).

As mentioned above, the bG meter 100 is used by the patient 142 for recording, *inter alia,* insulin dosage readings and spot measured glucose levels. Such bG data obtained by the bG meter 100 in one embodiment is transferable via the communication interface 124 to another electronic device, such the external device 132 (PC, PDA, or cellular telephone), or via the network 136 to the remote PC and/or HCP computer 134. Examples of such bG meters include but are not limited to, the Accu-Chek Active meter and the Accu-Chek Aviva system both by Roche Diagnostics, Inc., which are compatible with the Accu-Chek 360° Diabetes management software to download test results to a personal computer or the Accu-Chek Pocket Compass Software for downloading and communication with a PDA. The program may run on a remote server and generate result. The result is available by one or more communication mode(s) stated earlier. The program device is also functional with 3rd party devices which communicate with 132, 134. Examples of communicating and exchanging information between various devices is further provided in more detail in commonly owned U.S. Application Ser. No. 12/119,143.

Accordingly, it is to be appreciated that the bG meter 100 includes the software and hardware necessary to process, analyze and interpret the self-recorded diabetes patient (i.e., bG) data in accordance with predefined flow sequences (as described below in detail) and generate an appropriate data interpretation output. In one embodiment, the results of the data analysis and interpretation performed upon the stored patient data by the bG meter 100 are displayed in the form of a report, trend-monitoring graphs, and charts to help patients manage their physiological condition and support patient-doctor communications. In other embodiments, the bG data from the bG meter 100 may be used to generated reports (hardcopy or electronic) via the external device 132 and/or personal computer (PC) and/or HCP computer 134.

The bG meter 100 further provides the user and/or his or her HCP with the possibilities of a) editing data descriptions, e.g., the title and description of a record; b) saving records at a specified location, in particular in user-definable directories as described above; c) recalling records for display; d) searching records according to different criteria (date, time, title, description, context information, data indexing, time range, etc.); e) sorting records according to different criteria (values of the bG level, date, time, duration, title, description etc.); f) deleting records; g) exporting records; and/or h) performing data comparisons, modifying records, excluding records as is well known. Alternatively or additionally, these functions may be performed on an external device 132, a HCP computer 134 or an external server 135.

As used herein, lifestyle is described in general as a pattern in an individual's habits such as meals, exercise, and work schedule. The individual additionally may be on medications such as insulin therapy or orals that they are required to take in a periodic fashion. Influence of such action on glucose is implicitly considered by the present disclosure.

*Estimating True mean bG and HbA1C*

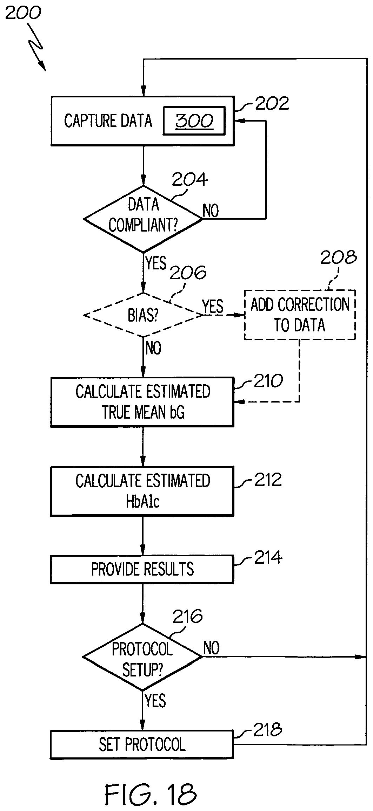

With reference made also to FIG. 18, a method 200 according to one embodiment of the present disclosure is described. In step 202, bG (i.e., spot) measurements of the patient 142 is captured. In one embodiment, each of the bG spot measurements is captured via strip 140 provided with a sample of the patient's which is then in turn read by a strip reader and analyzed by processor 102 to give the bG measurement of the patient 142. In other embodiments, the bG measurements may be captured at times dictated by the continuous glucose monitor 101 or other bG measuring devices and/or as commanded by the patient. As is well know the result of a newly taken bG measurement is displayed to the patient on display 108 as well as stored such as, for example, in memory 112 together with a time (e.g., GMT) and date of the measurement, via processor 102 reading clock 144 in step 202.

In one embodiment and generally, as mentioned above the bG meter 100 stores the results of the glucose (bG) measurements in its memory 112 together with a date-time stamp and associated event information (i.e., information regarding the context in which the measurement was obtained) to create a chronological sequence or set G of bG spot measurements, such as measurements $bG_1^k$, $bG_2^k$, $bG_3^k$, $bG_4^k$, and $bG_5^k$, where $k$ is the day. The measurement set G is sorted by increasing time and may span several days. In one embodiment, the date stored in memory with the measurement consists of some representation of day/month/year, and the time consists of some representation of the time of day (e.g. hh:mm:ss). In other embodiments, other date and time recording methods may be used, such as for example, using a Julian calendar and an alternative count interval for time.

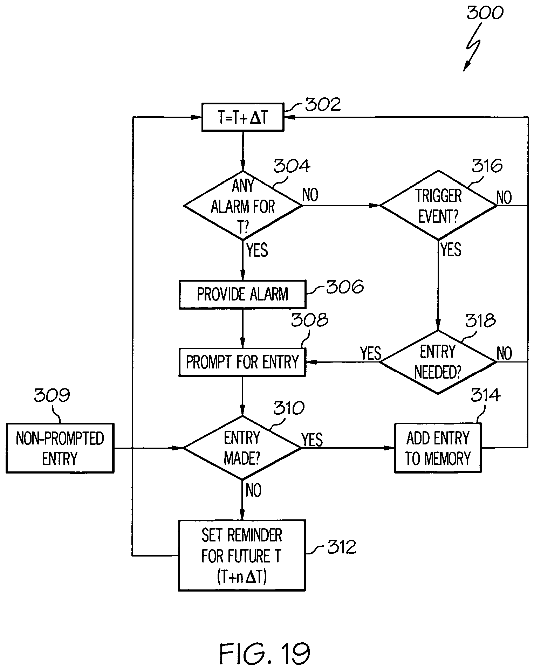

Along with each bG measurement, the patient is requested to input event information concerning the patient's lifestyle. In one embodiment, the bG meter 100 has enough memory to maintain bG data for at least 40-80 days with the associated event information concerning the patient's lifestyle. In another embodiment, the bG meter 100 has enough memory to maintain bG data for at least weeks with the associated event information concerning the patient's lifestyle. In one embodiment, lifestyle is classified by information concerning the following events: breakfast, lunch, supper, snack, exercise, physical activity, stress, alternate state, medication and optionally any other relevant event that is custom set into the meter. As with the bG measurements, such events are time stamped and associated with a description of event such as, for example, magnitude, intensity, duration, etc. Other such event characterizations are described more fully in commonly owned U.S. application Ser. Nos. 11/297,733 and 12/119,201. Manual input of the event description by the patient in one embodiment is driven by a questionnaire presented to the patient on the meter 100. In one embodiment, the questionnaire is provided by an HCP or designed to be set up by the patient according to provided instructions contained on the bG meter 100. In another embodiment, the meter 100 is provided with scheduled reminders (e.g., alarms for taking medication) which are provided at particular times in order to record such event information, e.g., via the questionnaire, within the compliance window according to the sampling schema of Table 7. An event scheduler 300 (FIG 19) may be provided for this purpose and executed by the processor 102 of the bG meter 100, which an example thereof is discussed hereafter. In some embodiments, the user may enter event information based on event triggers. For example, when a strip is inserted into the meter, the meter may prompt the user to enter event information.

FIG. 19 depicts a process of the event scheduler 300. In step 302, a timer T synced with the clock 144 is incremented wherein in step 304, the processor 102 checks a structured sampling schema, such as included in a protocol file provided in memory 110 or 112, to see whether the current time T matches an alarm time for inputting event information. It is to be appreciated that the structured sampling schema provides daily times (and hence alarms) for such collections. Also, ΔT can be periodic or determined by another algorithm where the algorithm determines ΔT dynamically so as to meet Table 7 requirements. If so, then in step 306 the processor 102 provides an alarm, such as an audio signal via speaker 148, visual signal via display 108, tactile signal (e.g. vibrations), email message, SMS, etc. to the patient 142. In step 308, after the patient 142 acknowledges the alarm via use of the user interface 146, insertion of a strip 140 into the strip reader 138, or after some set period of time, such as via expiration of a count down timer, the processor prompts the patient 142 for entry of the event information, via the questionnaire displayed on the display 108 by the processor 102.

In step 310, if the processor 102 fails to detect an entry via the user interface 146, after expiration of another count down timer e.g., 300 seconds (or in other embodiments the timer can range from few minutes to half an hour, and preferably 5 to 10 minutes), then the processor 102 in step 312 resets the alarm for a future time T which is still within the compliancy window of Table 7 for collecting the event information. If an entry was made and detected in step 310, such as placed into temporary memory, such as main memory 110 via the processor accepting input from the user interface 146, then in step 314 the processor 102 stores the entry in secondary memory 112 in a manner discussed previously above.

If in step 304, the structured sampling schema in memory 110 or 112 does not have an alarm for the processor 102, then the processor 102 in step 316 will check for any triggered events, e.g. auto initiated via another running process of the meter 100 or patient initiated via the user interface 146. If none is detected, then the processor 102 loops back to step 302 and the processes of the event scheduler 300 repeat. If a trigger event is detected, then in step 318 the processor 102 checks whether an entry is needed for the triggered event, such as by doing a lookup in the profile file. If an entry is needed then the process goes to step 308, and if not, the process loops back to step 302 and repeats. It is to be appreciated that the scheduler 300 when executed by the processor 102 of the meter 100 indicates and consequently records a SMBG measurement and the associated event information (e.g., via running the questionnaire) in compliance with the measurement schema provided according to Table 7. Any unforeseen event is also enterable into memory 112 of the meter 100 at any time by the patient 142 via manually running the questionnaire on the meter 100 e.g. a triggered event in step 312. For example, non-prompted entry may occur in step 309 whenever the user decides to submit an entry that was not directly prompted by an alarm.

Returning to FIG. 18, in step 204, the processor 102 checks to determine whether the bG data was collected in a compliant manner. Data compliant in step 204 means that rules and guidelines to collect data which were either programmatically or manually complied to by the user. In one embodiment, compliance check includes: checking to see whether the number of days in the bG data meets the minimum number needed to satisfy the nDay requirement (i.e., a predetermined period, which in one embodiment is >2 weeks if an HCP desires to use a few weeks of data to predict a future HbA1C and true mean bG in order to revise a patient's current therapy or behavior in order to try achieve their targeted goals, >80 days for a result having a CV <3%, or any amount of days there between for a snapshot); and checking to see whether a minimum number of the samples (nSample) collected at a requested sampling event per the collection schema (i.e., a predetermined amount, which in one preferred embodiment is >45, but in other embodiments may some other amount that is reason for a patient's lifestyle as determined by the HCP) also satisfy the sampling time window requirement (e.g., WinSize < 50 minutes). In one embodiment, the predetermined amount and period is read by the processor 102 from the structured sampling schema provided in memory. The result of the check on the collected bG data is either yes or no. In step 206, optionally, the processor 102 then checks for distribution of the time of bG samples with respect to the window center to see if there is a bias in the preferred time of post-prandial measurement. If there is, then in step 208 a correction for the bias may be added to the data. For example, if the time for bG measurement with respect to targeted post-prandial average time is biased then the algorithm will systematically alter the alert time in the future measurement of bG such as by alerting the patient later with respect to the targeted measurement time or may be raise an additional alert for measurement at a subsequent latter time and thus over period of days remove bias from the average time of measurement.

In another embodiment, an associated penalty in the precision of the estimated value may be flagged in step 208 such that a bias in time of bG measurement warning message is indicated with the provided results in step 214. After step 204, and optional step 206, if the bG data is compliant then the estimation processes Steps 210 and 212 are evoked. In step 210, the estimation process as mentioned previously above in reference to FIG. 7 bins together the data as per lifestyle as per event. The weighted mean bG is then determined by using the temporal weighting (harmonic weighting), whereby each of the weighted mean bG is then further time weighted by lifestyle related weight. The resulting value from step 210 is the estimate of the true mean bG value. Optionally, the estimated bG value is provided with an uncertainty window around the predicted value as is shown by FIGS. 16A-E. In step 212, the estimated HbA1C is then determined by solving the equation given in Table 7 using the estimated bG value from step 212, and the results then provided in step 214.

In another embodiment, an enhancement to the above model in Table 7 is to obtain an HbA1C value from an HbA1C assay, which can then be used as the patient specific intercept value c, instead of the given value of 0.5702. Such an embodiment is considered an estimated HbA1C with a one-point calibration. In still a further embodiment, another enhancement to the above model in Table 7, would be to obtain HbA1C using an HbA1C assay at two different points in time. These HbA1C values can then be used to determine a patient specific intercept value c and slope m. In such an embodiment, the two HbA1C values from the HbA1C assay not vary by more than $\pm 0.5$ % HbA1C to provide a good reliable slope m, assuming the assays are high quality (i.e., CV<2%). In another embodiment, the process 200 may then request whether the protocol file used for collection by the scheduler 300 needs updating in step 216. If so the protocol file is updated in step 218 via e.g., accepting user input via the user interface 146, e.g., from the processor 102 re-running a setup questionnaire on the display 108, receiving protocol changes from the HCP computer 134 when connected to the external device 132 such as, e.g., provided as a docking station, and combinations thereof. Afterwards, the process loops to step 202 and repeats. In another embodiment, Nathan's relation may be used for estimating HbA1c, wherein the estimated mean bG as described herein is used with Nathan's relation.

In still other embodiments, collection step 202, along with the event scheduler 300, is solely performed on the meter 100, wherein process steps 204-218 are performed on the HCP computer 134. In such an embodiment, the HCP computer 134 may also provide additional capabilities, such as using the collected bG data with other models to perform comparisons with the model results according to the present disclosure. For example, in one embodiment, the HCP could run the collected bG data through a HbA1C population based model derived from continuously monitored glucose data.

As previously mentioned above, in one embodiment the alerting and collecting by the processing of bG measurements and associated context of the bG measurement at the daily times and the events can be specified by the structured sampling schema that is stored in memory. In one embodiment, the daily times specified by the structured sampling schema are post-prandial times. In another embodiment, the daily times specified by the structured sampling schema are three post-prandial times and another time. In still another embodiment, the events specified by the structured sampling schema is a specific time with respect to start of a meal. In yet another embodiment, one of the events specified by the structured sampling schema is an aspect of glucose behavior related to the estimated true mean bG value, which in one embodiment, the aspect is a bG mean to peak value. In still another embodiment, the daily times specified by the structured sampling schema are at about 140 to about 240 minutes after a meal time. In a further embodiment, the daily times and the events specified by a structured sampling schema are tailored to a daily lifestyle pattern of the patient. In yet another embodiment, the daily times specified by the structured sampling schema range from about 140 to about 240 minutes after a meal time in accordance with the daily lifestyle pattern of the patient. In even yet another embodiment, such as that which can be used with the Type 2 patient population, a seven point measurement per day may be performed for three days. Such sets of measurements can be taken at regular time periods such as between every two or six weeks (such as every four weeks).

In another embodiment, the processor 102 is further programmed to weigh a bG measurement if collection of the bG measurement was within a time interval from the daily times specified by the structured sampling schema, whereby in one embodiment the time interval is at most ±50 minutes. It is to be appreciated that the time interval also captures the information of whether the measurements, which are typically performed by the patient at random times around a recommended time, are falling within or outside the time interval. Such information may be used by the processor 102 to evaluate whether the patient's lifestyle has been captured appropriately as reflected in the structured sampling schema and/or whether the patient requires training such as, for example, if a threshold number of measurement within the time interval is not meet over a period of time. For example, in one embodiment, if such a threshold number of measurements is not achieved, the processor 102 provides a message on the display 108 indicating a collection problem and can provide a recommendation, such as ways to improve collection compliancy. In still another embodiment, the processor 102 is further programmed to determine the estimated true mean bG value and the estimated HbA1C value from the weighted measurements of the collected bG measurements if a predetermined amount of the bG measurements per each of the daily times and the events has been collected. In one preferred embodiment, the predetermined amount is at least 80 days, and in another embodiment at least 60 days. In still another embodiment, the processor is further programmed to determine the estimated true mean bG value and the estimated HbA1C value from the weighted measurements of the collected bG measurements if the predetermined amount of the bG measurements per each of the daily times and the events has been collected, and if the collection of the bG measurements occurred within a predetermined period of at least 2 weeks.

*Displaying Grouped Estimated Biological Values or Grouped Predicted Biological Values*

As discussed above, while specific examples are presented herein of weighting bG measurements to determine mean bG values to then determine HbA1C values, other biological measurements can similarly be incorporated to estimate a patient's estimated (current) or predicted (future) condition based on the weighting of current and previous biological measurements. As used herein, "biological measurements" includes any type of measurement that provides insight into the patient's health with respect to diabetes. For example, biological measurements include, but are not limited to, bG measurements, HbA I C measurements, fructosamine, lipids, triglycerides, insulin concentration, etc. Accordingly, one or more of these biological measurements can be measured, weighted and used to determine an estimated or predicted biological measurement (either the same type of biological measurement that was obtained, or a different type of biological measurement, but one that can be determined from the type of biological measurement that was obtained). Moreover, the biological measurements can be grouped by one or more variables, such that the estimated or predicted biological values can be determined for each group in order to compare the effect each variable has on the patient's health.

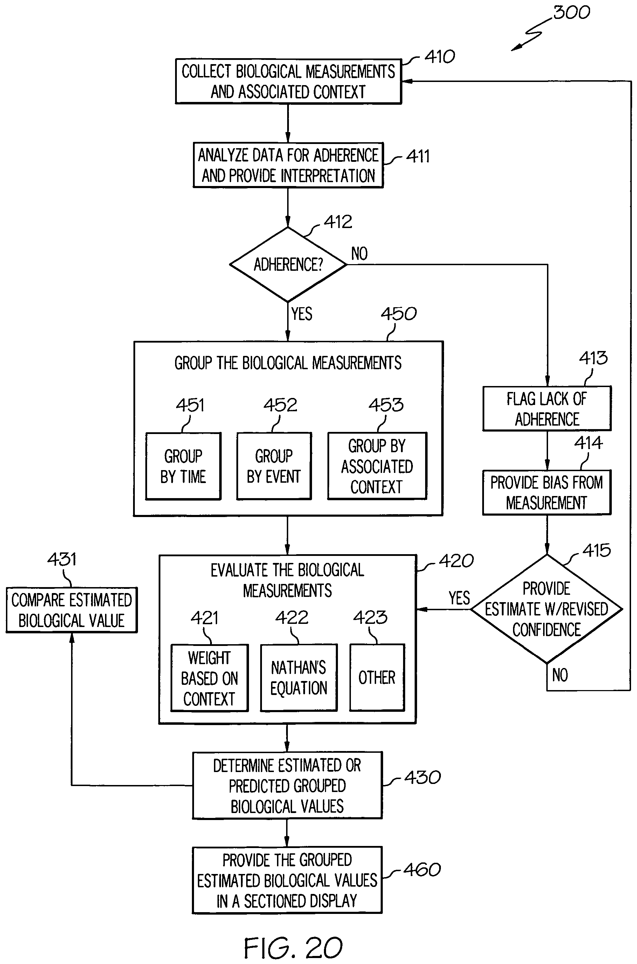

For example, referring now to FIG. 20, in some embodiments, the biological measurements collected comprise bG measurements, and the estimated/predicted values determined based on the weighted biological measurements comprise estimated HbA1C values. Therefore, the estimated HbA I C values determined from the weighted bG measurements as discussed above may be used in an informational delivery method 400 to provide grouped estimated HbA1C values in a sectioned display, i.e., a display comprising a plurality of sections. As used herein, "plurality of sections" refers to different areas on the display such that the estimated or predicted biological values for the different groups can be compared, as opposed to combined into a single value. For example, plurality of sections can include different sections of a pie graph, different adjacent bars on a bar graph, different plots on a graph, or any other format that allows for the comparison of estimated or predicted grouped biological values that are derived from the grouped biological measurements. Specifically, the estimated HbA1c values may be grouped by one or more variables (e.g., meal times, events, type of activity) so that a patient and/or physician may quickly assess the predicted impact different lifestyle components are contributing to the patient's health.

The informational delivery method 400 begins in step 410 by collecting biological measurements (e.g., bG measurements), and potentially other information such as associated context, in any manner discussed above. For example, the collection of bG measurements in step 410 can occur manually (e.g., using testing strips) or automatically (e.g., using a continuous bG meter) or simply comprise a transfer of previously obtained biological measurements, and potentially associated context, from a database (e.g., downloading the information from a bG meter to a physician's computer). In some embodiments, the collection of biological measurements may further be performed continuously or in accordance with a structured sampling schema wherein bG measurements were obtained at regulated times of the day such as within a prescribed period of time before and/or after meals, activities or other events. Such embodiments may help ensure the estimated or predicted biological values are obtained from a sufficient sample to reduce the effect of inconsistencies such as those that may occur when obtaining biological measurements at inconsistent times following meals or obtaining biological measurements arbitrarily throughout the day. In some embodiments, the structured sampling schema may comprise collecting measurements around a structured medication schema (i.e., collecting measurements as the patient takes a prescribed medication according to a structured medication schema). Furthermore, biological measurements may be collected in step 410 by a patient (e.g., self-testing), by a physician (e.g., laboratory testing) or by any third party.

Furthermore, the number of samples and the length of time in which the samples are obtained may be adjusted based on the patient. For example, in some embodiments, such as for patients having Type-2 diabetes, bG measurements may be obtained for 7-14 days before determining an estimated HbA1C value. In some embodiments, such as for patients having Type-1 diabetes, bG measurements may be obtained for 40 to 80 days. Other time period and measurement frequencies may alternatively be utilized to address the specific patients' lifestyle and pharmacological aspects or the specific problem being investigated.

In the invention, after or while the bG measurements (or other biological measurements) are collected in step 410, the data is analyzed for adherence in step 411 and an interpretation of the adherence is provided. Specifically, the data collected in step 410 is analyzed to determine whether the measurements were collected in a manner that complied with the testing protocol (e.g., whether measurements were taken at the proper times around an event within specified duration, whether measurements were taken for the proper number of days, whether the patient underwent any additional lifestyle changes that would influence the measurements, etc.). If the collected measurements were determined that they adhered to the testing protocol in step 412, then the bG measurements are evaluated in step 420.

If the measurements collected in step 410 are determined to not adhere in step 412 (such as missed measurements or measurements collected at incorrect times), then lack of adherence can be flagged in step 413. Specifically, the reason for lack of adherence can be presented to the patient, health care provider or any other relevant party so that any estimated values, if still determined, are reviewed with the lack of adherence in mind. In the invention, the bias from the measurements is provided in step 414 to the patient, health care provider, or other relevant party so that future measurements may be obtained to offset the bias from the measurements already collected. For example, if the patient routinely obtains measurements 30 minutes after they're supposed to, future measurements may be obtained 30 minutes before they were originally supposed to so that the bias of delayed measurements is counter balanced. Depending on the severity of the lack of adherence, the measurements may either still be used to determine estimated values with a revised level of confidence (i.e. accuracy) in step 415 and the collected data can be analyzed and interpreted.

Still referring to FIG. 20, the informational delivery method 400 further comprises grouping the biological measurements (e.g., bG measurements) in step 450 according to one or more set of variables. The one or more set of variables can comprise any time, event, associated context or other parameter that can be associated with the biological measurements such that grouping the biological measurements by the variables allows one to assess the impact of individual components on estimated or predicted biological values as will later be determined. For example, in some embodiments, the biological measurements may be grouped by time in step 451. Grouping by time can include grouping the biological measurements according to the time of the day in which the bG measurements were obtained. For example, the day may be broken down into a breakfast time interval ($T_{BF}$), a lunch interval ($T_{LU}$), a supper interval ($T_{SU}$), and a fasting interval ($T_{FA}$). Alternatively, grouping by time in step 451 can comprise grouping by certain weeks or parts of a week (e.g., weekends compared to weekdays), grouping by seasons (e.g., winter compared to fall), or grouping by any other relative time frame. These embodiments can be useful in comparing two time periods.

Still referring to FIG. 20, in some embodiments, grouping the biological measurements in step 450 comprises grouping the biological measurements by events in step 452. Grouping the biological measurements by event in step 452 can comprise grouping the biological measurements based on the occurrence of events associated with the bG measurements. Events can include the taking of a medication (e.g., whether any was taken, what type was taken, how much was taken), the performance of a physical activity (e.g., whether the patient exercises, what type of exercise was performed, how long did the patient exercise), the consumption of a particular type of food, or any other event that occurs in a patient's life which can be tracked (but still occurs with enough frequency so that variations on these events allow for the effective monitoring of their health). When events occur at the same time of the day, grouping by event 452 will essentially comprise grouping by time (i.e., the event and the time of the event are the same). However, when events occur at various times in a day, week, month or longer, grouping by the event will be distinct from grouping by regimented time frames. For example, where events consist of taking a medication, the biological measurements may be grouped based on the type of medication taken (or whether any medication was taken) and/or the amount of medication taken. Thus, one can group the biological measurements to appreciate the effect each event has on his or her estimated or predicted biological values to understand the relative impact (e.g., success) various medications, exercise regimens or other events have on his or her health.

In the invention, grouping the biological measurements in step 450 comprises grouping the biological measurements (e.g., bG measurements) by associated context in step 453. As discussed above, associated context can comprise variables on a patient's routine such as the size of a meal or the speed in which consumed food is digested. In such embodiments, not only are the biological measurements weighted by the associated context in step 420 to determine a more accurate estimated HbA1C value in step 430 (as will be discussed later herein), but the biological measurements collected in step 410 can be grouped by the same associated context such that the relative impact of variables within the associated context can be better appreciated.

The biological measurements may be grouped in step 450 automatically based on predetermined parameters (e.g., where a bG meter is programmed by default to group by time), or may be grouped based on the command of an operator. In some embodiments, the method may comprise prompting the operator to select the set of variables in which to group the biological measurements. For example, the operator may be prompted with multiple options such as time, events, associated context, or other variables which are available based on the known variables in which biological measurements were obtained. While specific examples have been provided of how biological measurements may be grouped in step 450 of informational delivery method 400, it should also be appreciated that the biological measurements may further be grouped by any other set of variables which may allow insight into their impact on the patient's estimated or predicted biological values.

Still referring to FIG. 20, the informational delivery method 400 further comprises evaluating the biological measurements (e.g., bG measurements) in step 420. Evaluating the biological measurements comprises interpreting the collected biological measurements using a selected protocol such that an estimated or predicted biological value can be determined in step 430. For example, in some embodiments, evaluating the biological measurements in step 420 comprises weighting the biological measurements based on associated context 421 as discussed above. In other embodiments, evaluating the biological measurements in step 420 comprises using Nathan's equation 422 with the collected measurements. In even other embodiments, evaluating the biological measurements in step 420 comprises using any other protocol in step 423 such that an estimated or predicted biological value can be determined in step 430. Evaluating the biological measurements in step 420 can be performed by any individual and/or machine, such as, for example, a computer, a bG meter and/or a personal digital assistant as discussed above. Once the biological measurements are evaluated, such as in accordance with one of the processes discussed above such that estimated true mean bG values can be determined, the estimated or predicted biological values (e.g., HbA1C values) are determined in step 430. The estimated/predicted biological values determined in step 430 may thus estimate or predict what the biological value (e.g., HbA1C level) will be for the patient as a result of his or her most recent biological measurement. For example, the patient and/or his or her physician may thus assess the relative impact of the estimated/predicted mean bG or HbA1c as a whole or as sectioned groups, and thereby appreciate the relative impact of the time, event and/or context associated with that bG measurement. Similar to evaluating the biological measurements in step 420, determining estimated or predicted biological values in step 430 may be performed by any individual and/or machine, such as, for example, a computer, a bG meter and/or a personal digital assistant.

In the invention, the estimated/predicted HbA1C values (or other estimated/predicted biological values) determined in step 430 are compared to the last measured actual HbA1C value (or other last measured biological value) in step 431. Comparing the estimated biological value with the actual biological value can provide the patient with the predicted increase or decrease in the HbA1C value as compared to their last actual measured HbA1C value. In these embodiments, the patient may then appreciate the effect their medication, lifestyle choices, etc. are having on their health. In some embodiments, the estimated biological value is compared to a target/reference biological value to appreciate the progress of the therapy. In some embodiments, the estimated/predicted biological value determined from one group of biological measurements is compared to the estimated/predicted value(s) determined from one or more other group(s) of biological measurements. In some embodiments, the estimated biological value determined for one time period can be compared to another biological value (either estimated or actual) from another time period. The reference values can be manually entered or can be retrieved from a storage device. For example, in some embodiments, the reference values are stored in a system such us a local system (e.g., the patient's bG monitor) or a remote system (e.g., a computer, server, or other storage device that can be accessed).

Still referring to FIG. 20, after the estimated or predicted biological values are determined in step 430, the grouped estimated HbA1C values are provided in a sectioned display in step 460. Specifically, the estimated or predicted biological values are provided such that each group is provided within at least one of a plurality of sections of the display. For example, where the biological values are grouped by time in step 451, the plurality of sections of the display will comprise sections for various time frames in which to display the respective group of estimated or predicted biological values. In some embodiments, the daily times in which bG measurements (or other biological measurements) were collected comprise a breakfast timeframe, a lunch timeframe, a supper timeframe and an overnight timeframe (e.g., a fasting timeframe). Similarly, the plurality of sections in the sectioned display could comprise a plurality of sections comprising a breakfast section, in which the grouped estimated or predicted biological values reflecting the impact of biological measurements taken during the breakfast timeframe are displayed, a lunch section, in which the grouped estimated or predicted biological values reflecting the impact of biological measurements taken during the lunch timeframe are displayed, a supper section, in which the grouped estimated or predicted biological values reflecting the impact of biological measurements taken during the supper timeframe are displayed, and an overnight section, in which the grouped estimated or predicted biological values reflecting the impact of biological measurements taken during the overnight timeframe are displayed.

Likewise, in embodiments where the estimated or predicted biological values are grouped by events in step 452, the plurality of sections in the sectioned display can, for example, comprise a first type of medication section, a second type of medication section, a no medication section, or the like. Additionally or alternatively, in some embodiments, the plurality of sections may comprise sections based on the amount or concentration of the medication. In even other embodiments, when the estimated or predicted biological values are grouped by other associated context variables in step 453, the plurality of sections of the sectioned display may be based off the different context variables (e.g., a large meal size section for estimated HbA1C values based on bG measurements taken after meals of a large size, a normal meal size section for estimated HbA1C values based on bG measurements taken after meals of a normal size, and a small meal size section for estimated HbA1C values based on bG measurements taken after meals of a small size). As such, the plurality of sections allows for the component-based display of grouped estimated or predicted biological values so that the effect of each component can be assessed. Optionally, an interpretation may be provided based on the grouped estimated or predicted values provided in the display conveying the relevant information such as the relative impact each component (i.e., variable) has on the patient, potential lifestyle changes, potential medication changes, etc.

Therefore, by grouping estimated or predicted biological values by events, one can quickly assess the relative success a prescribed therapy is having on the patient. For example, a patient, physician or other third-party can assess the relative impact attributed to the grouped estimated HbA1C values from each section of the sectioned display. Thus, when one section provides a greater impact to HbA1C values (such as when large meals account for the greatest impact on HbA1C values), its impact can quickly be visualized. This can be utilized to offer quick insight into the effectiveness of a therapy treatment (such as drug administration or exercise regimen) so that progress can be monitored. In addition, by allowing the quick assessment of the impact on the patient's HbA1C through the estimated HbA1C values, therapies can be quickly adjusted or discontinued (or lifestyles can be modified when possible) if they are not producing the expected or necessary results such as elevated glycemia or causing hypo glycemia. For example, in some embodiments, such as where the estimated HbA1C values are grouped based on events in which a new drug was and was not administered, the sectioned display can provide the effect on the patient's HbAIC when the drug was and was not administered. If administration of the drug produced little or no effect, the patient may adjust the drug amount, change the type of drug or stop the therapy to reduce unnecessary costs. The informational delivery method 400 allows this adjustment to occur in real time without waiting to collect actual HbA1C values for the patient during the next clinical visit.

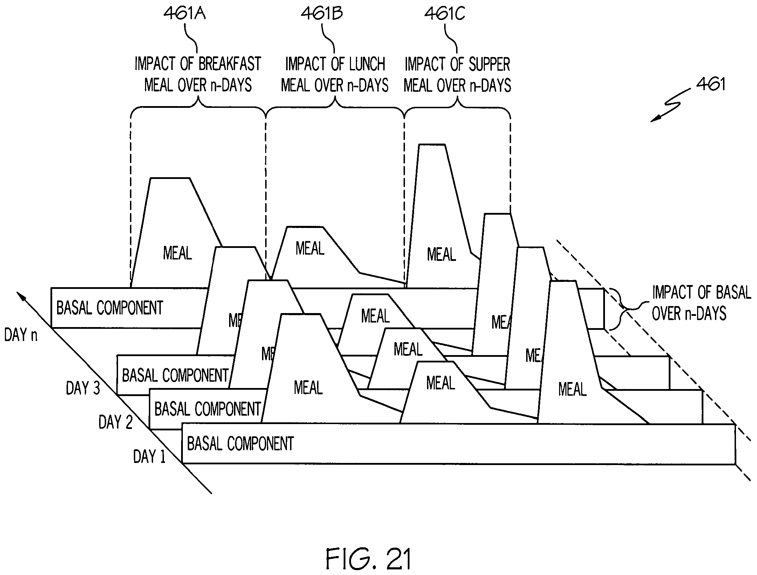

Referring now to FIGS. 20 and 21, an exemplary graphical visualization 461 is illustrated demonstrating the display of grouped biological measurements (such as HbA1C values) in accordance with step 450 of informational delivery method 400. Specifically, the exemplary visualization displays the biological measurements for each meal he or she has for multiple days grouped by the respective meal (i.e., breakfast, lunch and supper). Specifically, as illustrated in FIG. 21, the biological measurements are grouped into the first meal of each day 461 A (i.e., breakfast) the second meal of each day 461B (i.e., lunch), and the third meal of each day 461C (i.e., supper). By grouping these relative impacts, the patient and health care provider can better understand the relative impact each component has to his or her breakdown of glucose excursion.

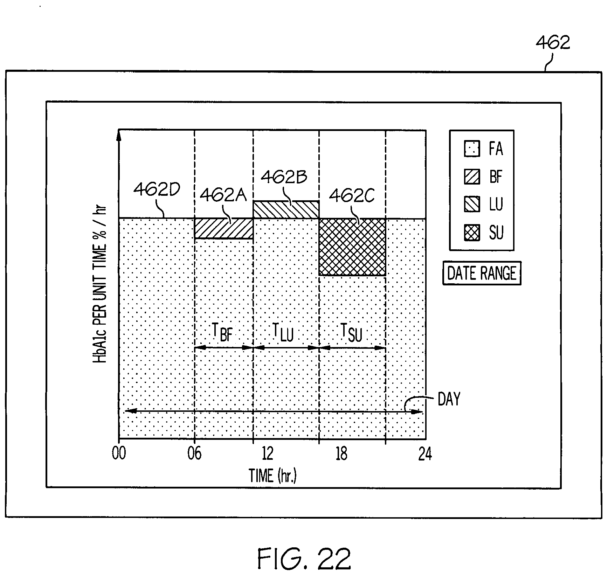

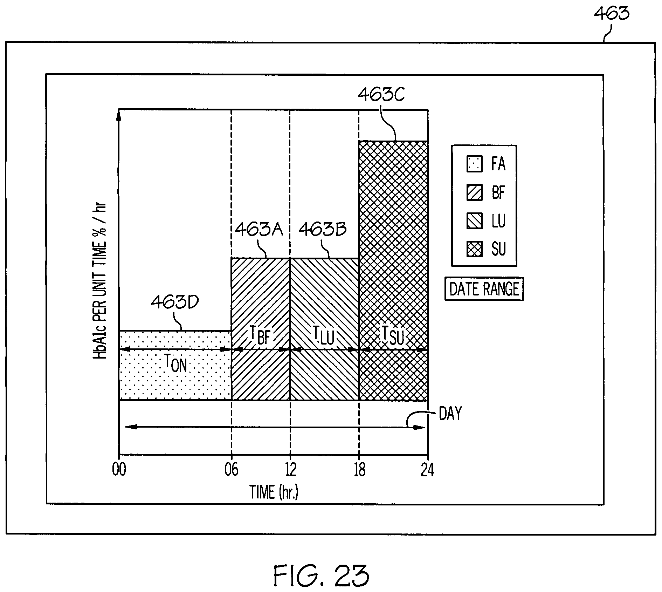

Referring now to FIGS. 20 and 22, a first exemplary sectioned display 462 is illustrated in which the contribution due to meal intake and insulin control action is displayed. In the first exemplary sectioned display 462, the bG measurements were grouped in step 450 according to the time of the day (i.e., a timeframe for breakfast $T_{BF}$, a timeframe for lunch $T_{LU}$, a timeframe for supper $T_{SU}$ and a timeframe for fasting) and the estimated values were determined as described by Equation (29B) and Equation (30A) to show relative effect of meals with respect to a fasting state. In some embodiments, other comparative interpretations may alternatively or additionally be provided, such as providing absolute values (as illustrated in FIG. 23), median values, mode values, etc. The grouped estimated HbAIC values are then provided in step 460 in the sectioned display by comparing the contribution due to the meal intake as well as the insulin control action with respect to the basal (i.e., the fasting contribution). Specifically, the height and width of each section of the section display is generated as follows: the X-axis represents a 24 hr day, the width of the section is proportional to the duration of the section, the height is the value of HbA1c for the section divided by the width of the section. The area of the section represents the HbA1c value, the width of the section has units of hours and the height has the unit of HbA1C% / hour. As illustrated, the contribution from breakfast 462A and supper 462C is overly controlled with respect to the basal 462D, while the contribution of lunch 462B shows positive contribution to the A1c. By providing this first exemplary sectioned display in step 460 of informational delivery method 400, the patient, physician or any other party may quickly assess the relative impact of each meal using estimated HbA1C values to develop a revised therapeutic plan to address the relative contributions without waiting for new clinical HbA1C measurements.

Referring now to FIGS. 20 and 23, a second exemplary sectioned display 463 is illustrated in which each group is displayed in its own section 463A-D as an individual component independent of one another. Similar to the first exemplary sectioned display 462 of FIG. 22, the bG measurements were grouped in step 450 according to the time of the day (i.e., a timeframe for breakfast $T_{BF}$, a timeframe for lunch $T_{LU}$, a timeframe for supper $T_{SU}$ and a timeframe for fasting) and the estimated HbA1C value for each group was determined so that the absolute values for each group are presented with respect to time so that the relative effect of each group is compared. The area of each section represents the respective contribution of HbA1c. The overall estimated HbA1c value can be displayed optionally along with the contribution by each of the section as HbA1c % value. Alternatively the values may be displayed in ratios or percentage of the overall HbA1c % value. The grouped estimated HbA1C values are then provided in step 460 in the sectioned display by displaying the contribution of each group independent of the others. As a result, the patient, physician or any other party can quickly assess the impact each section has (e.g., identifying that the supper section 463C contributes the greatest impact, as opposed to the breakfast section 463A or the lunch section 463B, on the basal 462D) and adjust the patient's therapy and/or lifestyle accordingly.

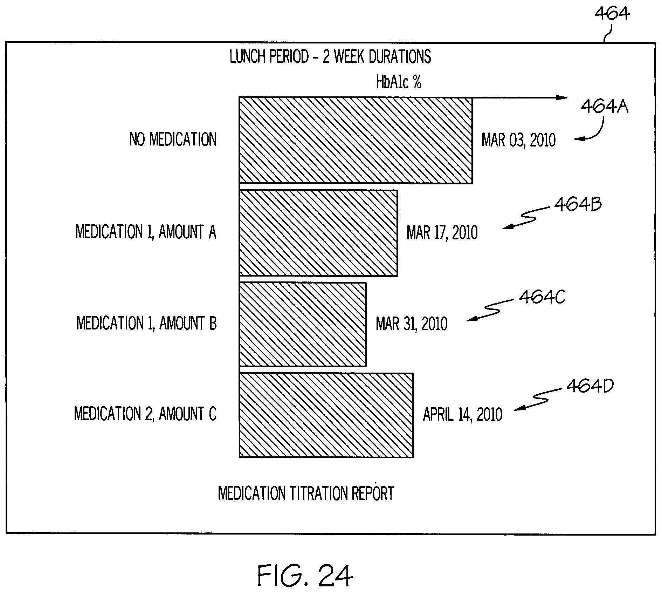

Referring now to FIGS. 20 and 24, a third exemplary sectioned display 464 is illustrated in which each section 464A-D is displayed to ascertain the relative impact of different types and amounts of medication. For the third exemplary sectioned display 464, the estimated/predicted HbA1C values were determined after the bG measurements were grouped in step 450 based on the time period in which a particular amount and type of medication was taken. Specifically, the estimated/predicted HbA1C values were grouped by a first two week time period in which no medication was taken 464A, a second two week time period (this 2 week period can be more or less) in which an "A" amount of "Medication 1" was taken 464B, a third time period in which a "B" amount of "Medication 2" was taken 464C, and a fourth time period in which a "C" amount of "Medication 3" was taken 464D. The estimated/predicted HbA1C values grouped in step 450 are then provided in step 460 in the sectioned display wherein a section corresponds to each time period (i.e., 464A, 464B, 464C, and 464D). As such, the relative impact of the patient's HbA1C can be assessed with respect to each group determine the relative success of each therapy (e.g., the effectiveness in reducing HbA1c/controlling glycemic excursion) of the different medication regimens.

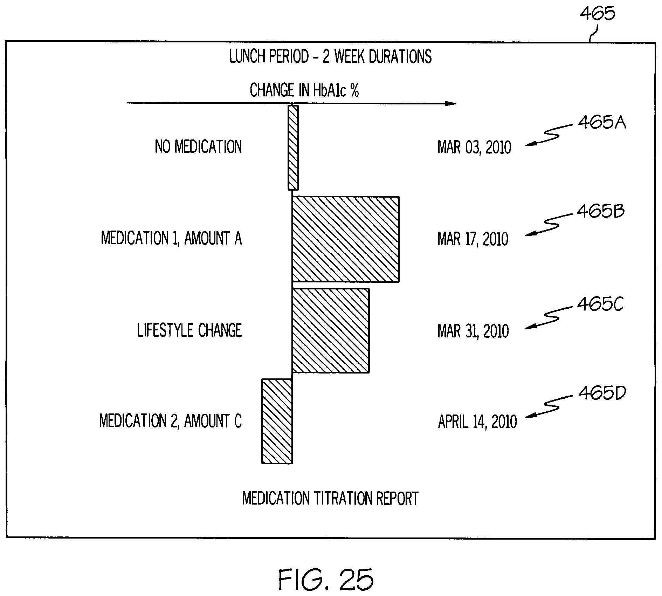

Referring now to FIGS. 20 and 25, a fourth exemplary sectioned display 465 is illustrated in which each section 465A-D is displayed to ascertain the relative impact of different types and amounts of medication and a lifestyle change (e.g., increased exercise or healthier eating). For the fourth exemplary sectioned display 465, the estimated/predicted change in HbA 1C values were determined after the bG measurements were grouped in step 450 based on the time period in which a particular event was occurring, wherein the event consisted of taking a medication, taking no medication, or a lifestyle change. Specifically, the estimated/predict change in HbA 1C values were grouped by a first two week time period in which no medication was taken 465A, a second two week time period in which an "A" amount of "Medication I" was taken 465B, a third time period in which the patient underwent a lifestyle change 465C, and a fourth time period in which a "C" amount of "Medication 2" was taken 465D. The estimated/predict change in HbA 1C values grouped in step 450 are then provided in step 460 in the sectioned display such that the impact of the second 465B, third 465C and fourth time frames 465D are illustrated compared to the impact of the first time frame 465A (i.e., when no medication was taken). As such, the patient, physician or any other party can quickly assess the impact of "Medication I, Amount A," "Lifestyle Change," and "Medication 2, Amount C" compared to that of "No Medication." The patient may then select or adjust a therapy based on the relative impact of each component. The change in HbA1c % is with reference to the case of no medication, or alternatively, to the selected reference type.

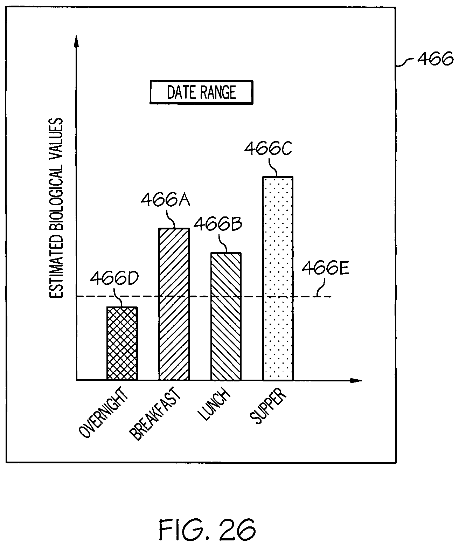

Referring now to FIGS. 20 and 26, a fifth exemplary sectioned display 466 is illustrated in which each section 466A-D is displayed to see how its current or previous estimated values (corresponding to the groups of breakfast 466A, lunch 466B, supper 466C and overnight (i.e., fasting) 466D), compares to the current or previous estimated values of other sections (which correlates with Equation (30) presented above in that the fasting component of Equation (30) is presented as the overnight section). For the fifth exemplary sectioned display 466, biological measurements were obtained (e.g., bG measurements) and weighted such that estimated biological values (e.g., predicted bG measurements or predicted HbA 1C values) could be determined. Prior to determining the estimated biological values, the biological measurements were grouped into the four groups identified on the x-axis. Comparative values from each group (e.g., averages, absolute values, etc.) are thereby displayed in their respective sections so the relative impact of each group can be visualized. For example, the patient can determine the greatest impact is coming from the supper group and therefore they may be especially cognizant of the food they eat, associated medication and activities they undergo around supper. The y-axis may comprise different values depending on the type of estimated biological values. For example, where the estimated biological values comprise HbA1C, the y-axis may be defined in HbA1C%, mmol/mol or mg/dL. Furthermore, a reference target line 466E may also be displayed across the fifth exemplary sectioned display 466 corresponding to the target overall levels for the patient (or any other relevant target value or reference value) to ascertain where the estimated values project for the patient with respect to their targeted or previous measurements.

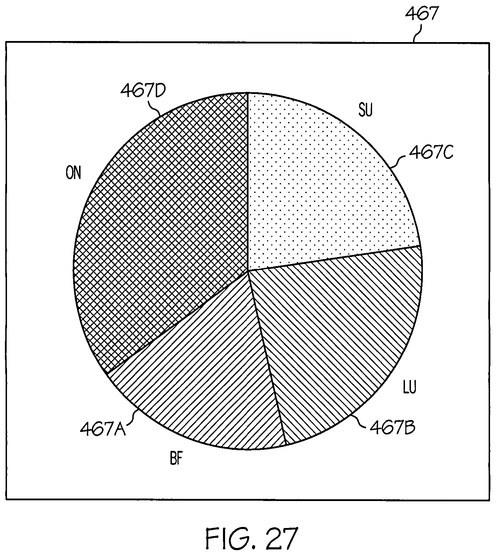

Referring now to FIGS. 20 and 27, a sixth exemplary sectioned display 467 is illustrated in which each section 467A-D is displayed in a pie chart to see how its estimated values (corresponding to the groups of breakfast 467A, lunch 467B, supper 467C and overnight (i.e., fasting) 467D), compares to the estimated values of other sections. For the sixth exemplary sectioned display 467, biological measurements were obtained (e.g., bG measurements) and weighted such that estimated biological values (e.g., predicted bG measurements or predicted HbA 1C values) could be determined. Prior to determining the estimated biological values, the biological measurements were grouped into the four groups identified on the perimeter of the pie chart. Comparative values from each group (e.g., averages, absolute values, etc.) are thereby displayed in their respective sections so the relative impact of each group can be visualized.

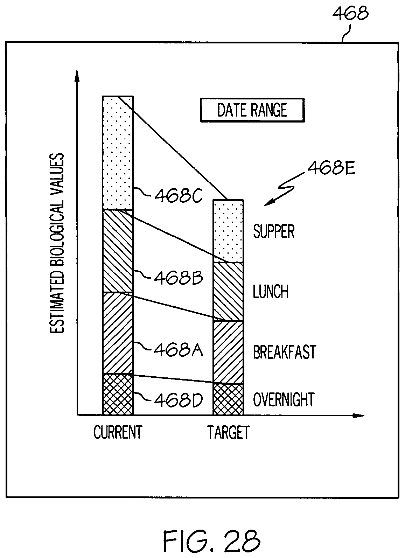

Referring now to FIGS. 20 and 28, a seventh exemplary sectioned display 468 is illustrated in which each section 468A-D is displayed to see how its estimated values (corresponding to the groups of breakfast 468A, lunch 468B, supper 468C and overnight (i.e., fasting) 468D), compares to the estimated values of other sections as well as target values. For the seventh exemplary sectioned display 468, biological measurements were obtained (e.g., bG measurements) and weighted such that estimated biological values (e.g., predicted bG measurements or predicted HbA1C values) could be determined. Prior to determining the estimated biological values, the biological measurements were grouped into the four groups identified along the bar chart. Comparative values from each group (e.g., averages, absolute values, etc.) are thereby displayed in their respective sections so the relative impact of each group can be visualized as an amount of the total biological value. The y-axis may comprise different values depending on the type of estimated/predicted biological values. For example, where the estimated/predicted biological values comprise HbA 1C, the y-axis may be defined in HbA1C%, mmol/mol or mg/dL. Furthermore, a reference target bar 468E may also be displayed adjacent the estimated/predicted biological values corresponding to the target levels for each section to ascertain where the estimated/predicted values project for the patient with respect to their target values. Additional indicia such as "Hi", "In Range", or "Low" may also be displayed adjacent each section to indicate the patient's status compared to their target levels.

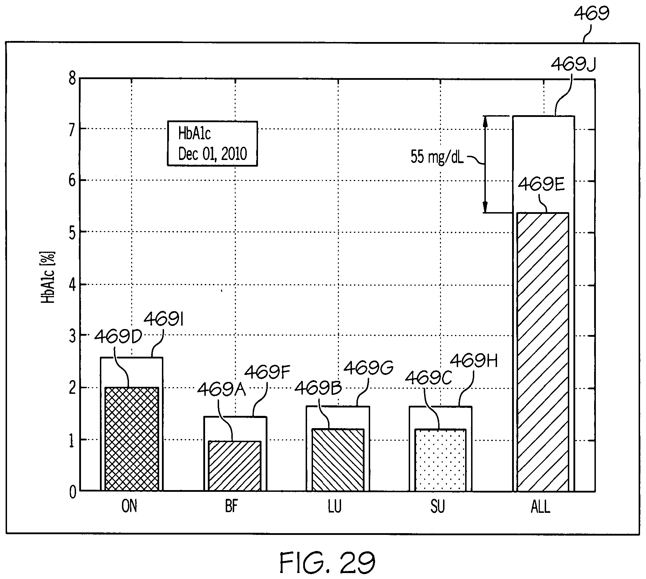

Referring now to FIGS. 20 and 29, an eighth exemplary sectioned display 469 is illustrated in which each section 469A-E is displayed to see how its estimated values (corresponding to the groups of breakfast 469A, lunch 469B, supper 469C, overnight (i.e., fasting) 469D, and all groups (i.e., total) 469E), compares to the estimated values of other sections as well as target values. For the eighth exemplary sectioned display 469, bG measurements were obtained and weighted such that estimated HbA 1C values could be determined. Prior to determining the estimated biological values, the biological measurements were grouped into the groups identified along the bar chart. Comparative values from each group (e.g., averages, absolute values, etc.) are thereby displayed in their respective sections so the relative impact of each group can be visualized. Furthermore, reference target bars 469F-J may also be displayed adjacent the estimated biological values corresponding to the target levels for each section to ascertain where the estimated values project for the patient with respect to their target values.

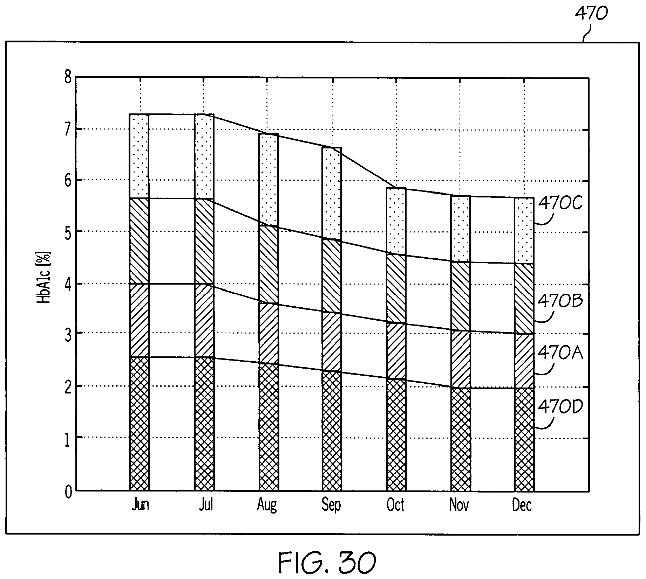

Referring now to FIGS. 20 and 30, a ninth exemplary sectioned display 470 is illustrated in which each section is displayed similar to seventh exemplary sectioned display 468 of FIG. 8, but wherein the biological measurements were grouped by month so that the patient's progress can be monitored. Specifically, the HbA1C% is illustrated for sections 470A-D (which can correspond to groups such as breakfast, lunch, supper and overnight (i.e., fasting)) and displayed in bar graph format for each month so that the patient's overall change by each month is visualized. Alternatively, the sections 470A-D may be grouped and displayed by weeks, seasons or any other temporal relationship.

While specific exemplary displays have been presented herein, it should be appreciated that other additional or alternative features may also be included. For example, such displays may selectively display additional information (such as the date range of measurements, labels/icons corresponding to the groups/events, etc), may be interactive (wherein the user can selectively change the data range, events, or other parameters that are displayed), may display actual values within each section of the sectioned display, may dynamically only display sections that are outside of target ranges, may be in color, gray scale or black and white, and/or contain any other relevant features for displaying grouped estimated biological values or grouped predicted biological values.

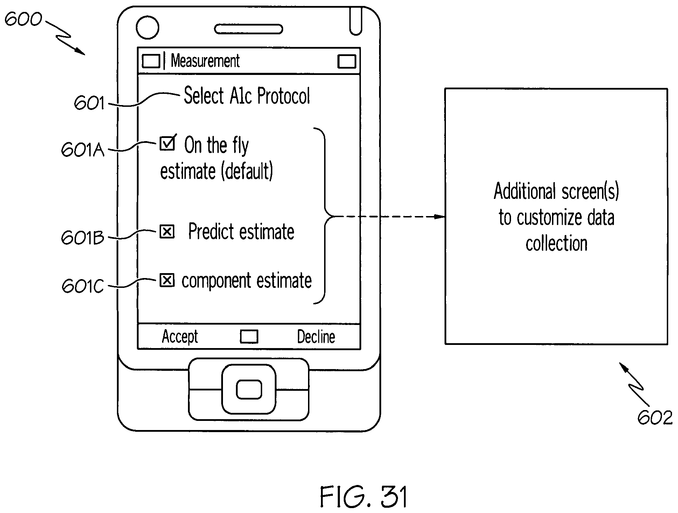

Referring now to FIG. 31, an exemplary textual screen 601 on an electronic device 600 (e.g., PDA, computer, etc.) is illustrated for prompting and conveying detailed information regarding the biological measurements and/or the estimated or predicted biological values presented in the exemplary displays discussed herein. Specifically, in some embodiments, after the user is presented their grouped estimated and/or predicted biological values in a sectioned display, they may be prompted or may request for details about various details such as, for example, the collected biological measurements, the method in which the estimated or predicted biological values were determined, how the estimated or predicted biological values were grouped, analysis constraints and/or the quality of results. For example, a user may desire to investigate the specific measurements that contributed to a display showing that their dinner-time estimated biological value is higher than desired. Therefore, the user may select that group (such as by selecting that section of the sectioned display, or following on-screen prompts to select that group) so that they may investigate the details of where that dinner-time measurements came from (i.e., what the measurements were, when they were recorded, what context was recorded relevant to those measurements, etc.).

In some embodiments, such as that illustrated in FIG. 31, the user may select or view what protocol is used to determine the estimated or prediction biological values (such as the HbA1C measurements as illustrated). For example, a plurality of protocols 601A, 601B, and 601C may be presented corresponding to different ways to determine estimated or predicted biological values (e.g., HbA 1C values) based on biological measurements (e.g., bG measurements). In some embodiments, these selection screens can lead to additional selection screen 602 that allow for the further customization (or further analysis) of what is displayed. In other embodiments, the exemplary textual display 601 may be presented prior to displaying the sectioned display. In these embodiments, the exemplary textual display 601 may prompt the user regarding the collection of biological measurements (e.g., when to collect, how many were collected, how many more need to be collected, etc.), the protocol to determine the estimated or predicted biological values (e.g., weighting based on associated context, Nathan's relation, etc.), or the variables by which to group the estimated or predicted biological values (e.g., time, event, etc.).

Furthermore, while specific examples have been presented in grouping estimated biological values or predicted biological values (such as estimated HbA 1C values according to step 450 of informational delivery method 400) and providing the grouped estimated biological values or grouped predicted biological values in a sectioned display (according to step 460 of informational delivery method 400), it should be appreciated that grouping may alternatively or additionally be performed in any other component-based methodology and be provided in any plurality of sections in the sectioned display that allows for the interpretation of the impact of each component.

Referring now to FIGS. 17 and 20, the informational delivery method 400 may be incorporated into a sectioned display device such as a bG meter 100 or similar electronic device comprising at least a display 108, an input terminal (such as a communications interface 124), memory (such as main memory or secondary memory 112) and a processor 102. In such embodiments, the input terminal (such as communication interface 124) collects both bG measurements and associated context of the bG measurements at daily times or events in accordance with step 410 of informational delivery method 400. The memory (such as main memory or secondary memory 112) stores the bG measurements, the associated context of the bG measurements and instructions. The processor 102 is in communication with the memory and operable to execute the instructions stored in the memory. Specifically, the instructions cause the processor to weight the bG measurements based on the associated context in accordance with step 420 of informational delivery method 400 and group the estimated HbA 1C values based on a set of variables in accordance with step 450 of informational delivery method 400. The instructions further cause the processor to provide the grouped estimated HbA 1C values in the display 108, such that the grouped estimated HbA 1C values are each displayed within a plurality of sections.

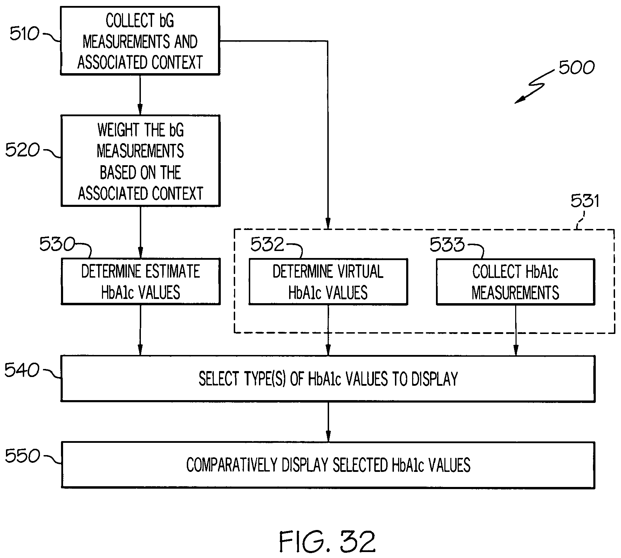

Referring now to FIG. 32, the estimated HbA1C values determined from mean bG values (or other estimated biological values determined from measured biological values) as discussed herein can be incorporated into a selective display method 500 in which one or more types of HbA 1C values (e.g., actual, estimated, virtual) can be selectively displayed, such as in a comparative format. As illustrated in FIG. 32, the selective display method 500 comprises collecting bG measurements and associated context in step 510 (similar to collecting measurements and associated context in step 410 of informational delivery method 400 illustrated in FIG. 20). After bG measurements are collected in step 510, the bG measurements are weighted based on the associated context in step 520 of the selective display method 500 (similar to weighting the bG measurements on associated context in step 420 of informational delivery method 400 illustrated in FIG. 20). The selective display method 500 then comprises determining estimated HbA1C values in step 530 as well as additional types of HbA1C values in step 531. Specifically, estimated HbA1C values can be determined in step 530 using the weighted bG measurements as discussed herein.

Additional HbA 1C values determined in step 531 can comprise any other HbA I C value actually measured from a patient or otherwise calculated from a patient based on other measurements such as bG measurements. For example, in some embodiments, an additional HbA 1C value determined in step 531 can comprise a virtual HbA 1C value determined in step 532. Virtual HbA 1C values are those determined purely as a function of glucose concentration in which it as assumed that the glycation process is the same for each patient (and wherein bG values are not weighted based on the specific context of the patient). As such, while the patient specific physiological variability is not directly addressed in determining the value, glycemic control within patients can nonetheless be compared. Virtual HbA1C values may be determined in step 532 based on bG measurements collected in step 510. In some embodiments, an additional HbA1C value determined in step 531 can comprise the patient's actual HbA1C value as previously or currently measured in a clinical setting. In such embodiments, determining an additional type of HbA1c values may thereby simply comprise collecting actual HbA1C measurements in step 533 such that collecting the actual HbA1C measurements can comprise downloading a set of measurements or continuously updating a set of measurements as new values are determined.

After various HbA1C values are determined or collected through steps 530 and 531 of the selective display method 500, the types of HbA1C values to display are selected in step 540. Specifically, one can select any or all HbA1C values to display such that they can compare the different HbA1C values of the patient. For example, in some embodiments, the method may be incorporated in an electronic device such as a bG meter, PDA or computer. The operator may then be prompted to select which values to compare based on the different values determined/obtained in steps 530 and 531. For example, the operator may be able to select estimated HbA 1C values (determined in step 530 which predicts the patient's HbA1C values based on previous bG measurements), actual HbA1C measurements (collected in step 533 which contains recent values actually measured from the patient) and/or virtual HbA1C values (determined in step 532 which estimates the patient's HbA1C value based on bG measurements, but relies on a population based model as opposed to weighting the individual bG measurements based on context). Furthermore, the user may also select the date range, events, or other parameter for which the HbA1C values are to be displayed.

After the types of HbA 1C values are selected in step 540, the selected types of HbA1C values are displayed in step 550 of the selective display method 500. Specifically, the selected types of HbA1C values are displayed such that a user can see and/or compare the various HbA1C values. In some embodiments, the various values may be plotted on a common graph. For example, where estimated HbA1C values and actual HbA1C values are selected, then both sets of values may be plotted so a patient can visualize how his or her estimated HbA1C compares to his or her previously measured actual HbA1C results. In some embodiments, the value of the selected types of HbA IC values are displayed such that the patient can see how new values (such as estimated HbA 1C values or virtual HbA1C values compare to previously measured actual HbA 1C values, such as by including the percent change. Such embodiments may allow the patient to more quickly assess the effect of new therapeutic regimens and determine how such lifestyle changes are influencing his or her health or what actions they can take to improve upon current trends.

In summary, the embodiments of the present disclosure address the ability to provide grouped estimated biological values or grouped predicted biological values (such as estimated or predicted HbA1C values) in a sectioned display to patients, physicians and/or any other party so that the effect of new treatment regimens or other variations in a patient's life can be more quickly assessed without waiting for clinical measurements of actual values. For example, by weighting obtained bG measurements, estimated HbA1C values may be determined through calculating true mean bG values. The bG measurements can be grouped on a set of variables so that the estimated HbA1C values may be displayed so that the relative impact of different times, events or other context can be examined. The grouped HbA1C values may thus be delivered in a sectioned display to quickly asses the effect of each variable-based component towards the patient's HbA1C. Additionally, estimated HbA1C values may be determined along with other types of HbA 1C values so that a user may select different types of HbA1C values for comparison. Such embodiments can allow for, among other things, a patient's previously measured actual HbA1C values to be compared with newly determined estimated HbA1C values to study the effect of recent therapeutic and/or lifestyle changes.

**Claims**

1. A sectioned display device for displaying grouped estimated biological values or grouped predicted biological values, comprising:

    - a sectioned display (108);
    - an input terminal (124) for collecting biological measurements in accordance with a structured sampling schema and for collecting associated context of the collected biological measurements;
    - memory (110) for storing the collected biological measurements and having stored instructions; and
    - a processor (102) in communication with the memory and operable to execute the instructions, the instructions causing the processor (102) to:

        - analyze whether the collected biological measurements adhere to the structured sampling schema;
        - if the collected biological measurements are determined to not adhere to the structured sampling schema, flag lack of adherence and provide a bias from the collected biological measurements so that future measurements may be obtained to offset the bias from already collected measurements;
        - if the collected biological measurements are determined to adhere to the structured sampling schema, group the biological measurements based on the set of variables, wherein the set of variables comprises the associated context associated with the biological measurements, wherein the grouping comprises grouping the biological measurements by the associated context, wherein the associated context comprises variables on a patient's routine;
        - evaluate the biological measurements to determine grouped estimated biological values or grouped predicted biological values, comprising weighting each of the collected biological measurements based on the associated context;
        - compare the grouped estimated biological values or grouped predicted biological values to a last measured biological value and provide a predicted increase or decrease in the grouped estimated biological values or grouped predicted biological values as compared to their last actual measured biological value; and
        - provide the grouped estimated biological values or grouped predicted biological values within a plurality of sections in the sectioned display (108), wherein the plurality of sections corresponds to the set of variables.

2. The sectioned display device of claim 1, wherein the biological measurements comprise bG measurements.

3. The sectioned display device of claim 1 or 2, wherein the estimated biological values comprise estimated or predicted glycated hemoglobin (HBAIC) values, fructosamine values, true mean blood glucose value, or triglyceride values.

4. The sectioned display device according to any one of the preceding claims, wherein the set of variables comprises a timeframe in which the collected biological measurement was obtained.

5. The sectioned display device of claim 4, wherein the timeframe comprises a breakfast timeframe, a lunch timeframe, a supper timeframe and an overnight timeframe.

6. The sectioned display device of claim 5, wherein the plurality of sections comprises:

   - a breakfast section in which the grouped estimated biological values or grouped predicted biological values reflecting the impact of biological measurements collected during the breakfast timeframe are displayed;
   - a lunch section in which the grouped estimated biological values or grouped predicted biological values reflecting the impact of biological measurements collected during the lunch timeframe are displayed;
   - a supper section in which the grouped estimated biological values or grouped predicted biological values reflecting the impact of biological measurements collected during the supper timeframe are displayed; and
   - a fasting section in which the grouped estimated biological values or grouped predicted biological values reflecting the impact of biological measurements collected during the overnight timeframe are displayed.

7. The sectioned display device according to any one of the preceding claims, wherein the set of variables comprises a type and/or amount of medication administered relative to the collected biological measurement, preferably wherein the plurality of sections comprises a first type of medication section and a second type of medication section.

8. The sectioned display device of claim 7, wherein the plurality of sections comprises a first amount of a first type of medication section and a second amount of the first type of medication section, wherein preferably the instructions further cause the processor (102) to evaluate an effect of the type and/or the amount of medication based on the grouped estimated biological values or grouped predicted biological values provided within the plurality of sections in the sectioned display (108).

9. The sectioned display device according to any of the preceding claims, wherein the instructions further cause the processor (102) to provide an interpretation of the grouped estimated biological values or grouped predicted biological values provided in the sectioned display (108).

10. The sectioned display device according to any of the preceding claims, wherein the sectioned display device comprises a bG meter and preferably the bG meter further comprises an alarm that prompts an operator to collect biological samples according to a structured sampling schema stored on the memory (110).

11. The sectioned display device according to any of the preceding claims, wherein the sectioned display device is configured to be in communication with an external server (135) that is configured to evaluate the biological measurements to determine the estimated or predicted biological values and/or wherein the instructions further cause the processor (102) to provide an interpretation of the grouped estimated biological values or grouped predicted biological values provided in the sectioned display (108).


**Patentansprüche**

1. Geteilte Anzeigevorrichtung zum Anzeigen zusammengefasster biologischer Schätzwerte oder zusammengefasster biologischer Prognosewerte, umfassend:

   - eine geteilte Anzeige (108);
   - ein Eingabeterminal (124) zum Sammeln biologischer Messungen gemäß einem strukturierten Probennahmeschema und zum Sammeln von zugehörigem Kontext der gesammelten biologischen Messungen;
   - einen Speicher (110) zum Speichern der gesammelten biologischen Messungen und mit gespeicherten Anweisungen; und
   - einen Prozessor (102) in Kommunikation mit dem Speicher und der so betrieben werden kann, dass er die Anweisungen ausführt, wobei die Anweisungen den Prozessor (102) zu Folgendem veranlassen:

      - Analysieren, ob sich die gesammelten biologischen Messungen an das strukturierte Probennahmeschema halten;
      - wenn bestimmt wird, dass sich die gesammelten biologischen Messungen nicht an das strukturierte Probennahmeschema halten, Markieren fehlender Einhaltung und Bereitstellen einer Abweichung von den gesammelten biologischen Messungen, sodass zukünftige Messungen erhalten werden können, um die Abweichung von bereits gesammelten Messungen auszugleichen;

- wenn bestimmt wird, dass sich die gesammelten biologischen Messungen an das strukturierte Probennahmeschema halten, Zusammenfassen der biologischen Messungen basierend auf dem Satz von Variablen, wobei der Satz von Variablen den zugehörigen Kontext in Verbindung mit den biologischen Messungen umfasst, wobei das Zusammenfassen das Zusammenfassen der biologischen Messungen mittels des zugehörigen Kontextes umfasst, wobei der zugehörige Kontext Variablen zu einer Patientenroutine umfasst;

- Auswerten der biologischen Messungen zum Bestimmen zusammengefasster biologischer Schätzwerte oder zusammengefasster biologischer Prognosewerte, umfassend ein Wichten jeder der gesammelten biologischen Messungen basierend auf dem zugehörigen Kontext;

- Vergleichen der zusammengefassten biologischen Schätzwerte oder zusammengefassten biologischen Prognosewerte mit einem letzten gemessenen biologischen Wert und Bereitstellen eines prognostizierten Anstiegs oder Abfalls der zusammengefassten biologischen Schätzwerte oder zusammengefassten biologischen Prognosewerte im Vergleich zu ihrem letzten tatsächlich gemessenen biologischen Wert; und

- Bereitstellen der zusammengefassten biologischen Schätzwerte oder zusammengefassten biologischen Prognosewerte innerhalb einer Vielzahl von Abschnitten in der geteilten Anzeige (108), wobei die Vielzahl von Abschnitten dem Satz von Variablen entspricht.

2. Geteilte Anzeigevorrichtung nach Anspruch 1, wobei die biologischen Messungen bG-Messungen umfassen.

3. Geteilte Anzeigevorrichtung nach Anspruch 1 oder 2, wobei die biologischen Schätzwerte geschätzte oder prognostizierte Werte bezüglich glykosyliertem Hämoglobin (HbA1C), Fructosamin-Werte, den wahren mittleren Blutzuckerwert oder Triglyceridwerte umfassen.

4. Geteilte Anzeigevorrichtung nach einem der vorstehenden Ansprüche, wobei der Satz von Variablen einen Zeitrahmen umfasst, in dem die gesammelte biologische Messung erhalten wurde.

5. Geteilte Anzeigevorrichtung nach Anspruch 4, wobei der Zeitrahmen einen Frühstückszeitrahmen, einen Mittagessenzeitrahmen, einen Abendessenzeitrahmen und einen Nachtzeitrahmen umfasst.

6. Geteilte Anzeigevorrichtung nach Anspruch 5, wobei die Vielzahl von Abschnitten Folgendes umfasst:

- einen Frühstücksabschnitt, in dem die zusammengefassten biologischen Schätzwerte oder zusammengefassten biologischen Prognosewerte angezeigt werden, die die Auswirkung biologischer Messungen, die während des Frühstückszeitrahmens gesammelt wurden, widerspiegeln;

- einen Mittagessenabschnitt, in dem die zusammengefassten biologischen Schätzwerte oder zusammengefassten biologischen Prognosewerte angezeigt werden, die die Auswirkung biologischer Messungen, die während des Mittagessenzeitrahmens gesammelt wurden, widerspiegeln;

- einen Abendessensabschnitt, in dem die zusammengefassten biologischen Schätzwerte oder zusammengefassten biologischen Prognosewerte angezeigt werden, die die Auswirkung biologischer Messungen, die während des Abendessenszeitrahmens gesammelt wurden, widerspiegeln; und

- einen Nüchtern-Abschnitt, in dem die zusammengefassten biologischen Schätzwerte oder zusammengefassten biologischen Prognosewerte angezeigt werden, die die Auswirkung biologischer Messungen, die während des Nachtzeitrahmens gesammelt wurden, widerspiegeln.

7. Geteilte Anzeigevorrichtung nach einem der vorstehenden Ansprüche, wobei der Satz von Variablen eine Art und/oder Menge einer verabreichten Medikation in Bezug auf die gesammelte biologische Messung umfasst, vorzugsweise wobei die Vielzahl von Abschnitten einen Abschnitt für eine erste Art von Medikation und einen Abschnitt für eine zweite Art von Medikation umfasst.

8. Geteilte Anzeigevorrichtung nach Anspruch 7, wobei die Vielzahl von Abschnitten einen Abschnitt für eine erste Menge einer ersten Art von Medikation und einen Abschnitt für eine zweite Menge der ersten Art von Medikation umfasst, wobei vorzugsweise die Anweisungen ferner den Prozessor (102) veranlassen, eine Wirkung der Art und/oder der Menge von Medikation basierend auf den zusammengefassten biologischen Schätzwerten oder zusammengefassten biologischen Prognosewerten, die innerhalb der Vielzahl von Abschnitten in der geteilten Anzeige (108) bereitgestellt werden, auszuwerten.

**EP 2 486 851 B1**

9. Geteilte Anzeigevorrichtung nach einem der vorstehenden Ansprüche, wobei die Anweisungen ferner den Prozessor (102) veranlassen, eine Auslegung der zusammengefassten biologischen Schätzwerte oder zusammengefassten biologischen Prognosewerte, die in der geteilten Anzeige (108) bereitgestellt werden, bereitzustellen.

10. Geteilte Anzeigevorrichtung nach einem der vorstehenden Ansprüche, wobei die geteilte Anzeigevorrichtung ein bG-Messgerät umfasst und vorzugsweise das bG-Messgerät ferner einen Alarm umfasst, der einen Bediener veranlasst, biologische Proben gemäß einem auf dem Speicher (110) gespeicherten strukturierten Probennahmeschema zu sammeln.

11. Geteilte Anzeigevorrichtung nach einem der vorstehenden Ansprüche, wobei die geteilte Anzeigevorrichtung für die Kommunikation mit einem externen Server (135) konfiguriert ist, der zum Auswerten der biologischen Messungen zum Bestimmen der geschätzten oder prognostizierten biologischen Werte konfiguriert ist, und/oder wobei die Anweisungen ferner den Prozessor (102) veranlassen, eine Auslegung der zusammengefassten biologischen Schätzwerte oder zusammengefassten biologischen Prognosewerte, die in der geteilten Anzeige (108) bereitgestellt werden, bereitzustellen.

**Revendications**

1. Dispositif d'affichage en sections pour l'affichage de valeurs biologiques estimées groupées ou de valeurs biologiques prédites groupées, comprenant :

   - un affichage en sections (108) ;
   - un terminal d'entrée (124) pour la collecte de mesures biologiques conformément à un schéma d'échantillonnage structuré et pour la collecte du contexte associé aux mesures biologiques collectées ;
   - une mémoire (110) pour le stockage des mesures biologiques collectées et ayant des instructions stockées ; et
   - un processeur (102) en communication avec la mémoire et pouvant fonctionner pour exécuter les instructions, les instructions amenant le processeur (102) à :

     - analyser si les mesures biologiques collectées adhèrent au schéma d'échantillonnage structuré ;
     - si les mesures biologiques collectées sont déterminées comme n'adhérant pas au schéma d'échantillonnage structuré, signaler le manque d'adhésion et fournir un biais à partir des mesures biologiques collectées de telle sorte que les futures mesures peuvent être obtenues pour compenser le biais de mesures déjà collectées ;
     - si les mesures biologiques collectées sont déterminées comme adhérant au schéma d'échantillonnage structuré, grouper les mesures biologiques sur la base de l'ensemble de variables, dans lequel l'ensemble de variables comprend le contexte associé aux mesures biologiques, dans lequel le regroupement comprend le regroupement des mesures biologiques par le contexte associé, dans lequel le contexte associé comprend les variables sur une routine de patient ;
     - évaluer les mesures biologiques pour déterminer les valeurs biologiques estimées groupées ou les valeurs biologiques prédites groupées, comprenant la pondération de chacune des mesures biologiques collectées sur la base du contexte associé ;
     - comparer les valeurs biologiques estimées groupées ou les valeurs biologiques prédites groupées à une dernière valeur biologique mesurée et fournir une augmentation ou une diminution prédite dans les valeurs biologiques estimées groupées ou les valeurs biologiques prédites groupées par comparaison à leur dernière valeur biologique mesurée réelle ; et
     - fournir les valeurs biologiques estimées groupées ou les valeurs biologiques prédites groupées au sein d'une pluralité de sections dans l'affichage en sections (108), dans lequel la pluralité de sections correspond à l'ensemble de variables.

2. Dispositif d'affichage en sections selon la revendication 1, dans lequel les mesures biologiques comprennent des mesures de glycémie.

3. Dispositif d'affichage en sections selon la revendication 1 ou 2, dans lequel les valeurs biologiques estimées comprennent des valeurs estimées ou prédites d'hémoglobine glyquée (HbA1C), des valeurs de fructosamine, une valeur de glycémie moyenne réelle ou des valeurs de triglycérides.

4. Dispositif d'affichage en sections selon l'une quelconque des revendications précédentes, dans lequel l'ensemble de variables comprend une fenêtre de temps dans laquelle la mesure biologique collectée a été obtenue.

5. Dispositif d'affichage en sections selon la revendication 4, dans lequel la fenêtre de temps comprend une fenêtre de temps de petit-déjeuner, une fenêtre de temps de déjeuner, une fenêtre de temps de dîner et une fenêtre de temps de nuit.

6. Dispositif d'affichage en sections selon la revendication 5, dans lequel la pluralité de sections comprend :

- une section petit-déjeuner dans laquelle les valeurs biologiques estimées groupées ou les valeurs biologiques prédites groupées reflétant l'impact des mesures biologiques collectées pendant la fenêtre de temps de petit-déjeuner sont affichées ;
- une section déjeuner dans laquelle les valeurs biologiques estimées groupées ou les valeurs biologiques prédites groupées reflétant l'impact des mesures biologiques collectées pendant la fenêtre de temps de déjeuner sont affichées ;
- une section dîner dans laquelle les valeurs biologiques estimées groupées ou les valeurs biologiques prédites groupées reflétant l'impact des mesures biologiques collectées pendant la fenêtre de temps de dîner sont affichées ; et
- une section jeûne dans laquelle les valeurs biologiques estimées groupées ou les valeurs biologiques prédites groupées reflétant l'impact des mesures biologiques collectées pendant la fenêtre de temps de nuit sont affichées.

7. Dispositif d'affichage en sections selon l'une quelconque des revendications précédentes, dans lequel l'ensemble de variables comprend un type et/ou une quantité de médicament administré en fonction de la mesure biologique collectée, de préférence dans lequel la pluralité de sections comprend une section de premier type de médicament et une section de second type de médicament.

8. Dispositif d'affichage en sections selon la revendication 7, dans lequel la pluralité de sections comprend une section de première quantité d'un premier type de médicament et une section de seconde quantité du premier type de médicament, dans lequel de préférence les instructions amènent en outre le processeur (102) à évaluer un effet du type et/ou de la quantité de médicament sur la base des valeurs biologiques estimées groupées ou des valeurs biologiques prédites groupées fournies au sein de la pluralité de sections dans l'affichage en sections (108).

9. Dispositif d'affichage en sections selon l'une quelconque des revendications précédentes, dans lequel les instructions amènent en outre le processeur (102) à fournir une interprétation des valeurs biologiques estimées groupées ou des valeurs biologiques prédites groupées fournies dans l'affichage en sections (108).

10. Dispositif d'affichage en sections selon l'une quelconque des revendications précédentes, dans lequel le dispositif d'affichage en sections comprend un glucomètre et de préférence le glucomètre comprend en outre une alarme qui invite un opérateur à collecter des échantillons biologiques selon un schéma d'échantillonnage structuré stocké sur la mémoire (110).

11. Dispositif d'affichage en sections selon l'une quelconque des revendications précédentes, dans lequel le dispositif d'affichage en sections est conçu pour être en communication avec un serveur externe (135) qui est configuré pour évaluer les mesures biologiques pour déterminer les valeurs biologiques estimées ou prédites et/ou dans lequel les instructions amènent en outre le processeur (102) à fournir une interprétation des valeurs biologiques estimées groupées ou des valeurs biologiques prédites groupées fournies dans l'affichage en sections (108).

FIG.1

FIG.5

FIG. 2

Glucose excursion due to meal

FIG. 3

EP 2 486 851 B1

FIG. 4

EP 2 486 851 B1

FIG. 6

EP 2 486 851 B1

FIG. 7

FIG. 8

EP 2 486 851 B1

CORRELATION COEFF. BETWEEN bG(t) & HbA1c

FIG. 9

EP 2 486 851 B1

FIG. 10

FIG. 11

EP 2 486 851 B1

FIG. 12

FIG. 13

FIG. 14

FIG.15C

FIG.15B

FIG.15E

FIG.15A

FIG.15D

52

FIG.16A

FIG.16B

FIG.16C

FIG.16D

FIG.16E

53

bG METER 100

102 PROCESSOR
104
MAIN MEMORY
110

140 STRIP
138 STRIP READER

112 SECONDARY MEMORY
114 HARD DISK DRIVE
116 REMOVABLE STORAGE DRIVE
122 INTERFACE

118 REMOVABLE STORAGE UNIT
120 REMOVABLE STORAGE UNIT

142 PATIENT
146 USER INTERFACE

101 MEASURING DEVICE
148 SPEAKER

124 COMMUNICATIONS INTERFACE
125
132 PC/PDA/ DOCKING STN.
130
136

103 INFUSION DEVICE
106 DISPLAY INTERFACE
144 CLOCK
150 POWER
128

108 DISPLAY

134 REMOTE PC/ HCP PC
135 EXTERNAL SERVER
130

FIG. 17

128

EP 2 486 851 B1

54

*200*

FIG. 18

FIG. 19

300

COLLECT BIOLOGICAL MEASUREMENTS
AND ASSOCIATED CONTEXT ⌐410

ANALYZE DATA FOR ADHERENCE ⌐411
AND PROVIDE INTERPRETATION

412

ADHERENCE? —— NO

YES

GROUP THE BIOLOGICAL MEASUREMENTS    450

451          452          453

GROUP     GROUP     GROUP BY
BY TIME   BY EVENT  ASSOCIATED
                    CONTEXT

FLAG LACK OF
ADHERENCE    413

PROVIDE BIAS FROM    414
MEASUREMENT

EVALUATE THE BIOLOGICAL    ⌐420
MEASUREMENTS

421        422        423

WEIGHT     NATHAN'S   OTHER
BASED ON   EQUATION
CONTEXT

415
PROVIDE
ESTIMATE W/REVISED
CONFIDENCE

YES          NO

COMPARE ESTIMATED    431
BIOLOGICAL VALUE

DETERMINE ESTIMATED OR
PREDICTED GROUPED    ⌐430
BIOLOGICAL VALUES

PROVIDE THE GROUPED
ESTIMATED BIOLOGICAL VALUES    ⌐460
IN A SECTIONED DISPLAY

FIG. 20

FIG. 21

EP 2 486 851 B1

462

FIG. 22

FIG. 23

FIG. 24

EP 2 486 851 B1

LUNCH PERIOD – 2 WEEK DURATIONS

CHANGE IN HbA1c %

NO MEDICATION — MAR 03, 2010 — 465A

MEDICATION 1, AMOUNT A — MAR 17, 2010 — 465B

LIFESTYLE CHANGE — MAR 31, 2010 — 465C

MEDICATION 2, AMOUNT C — APRIL 14, 2010 — 465D

MEDICATION TITRATION REPORT

465

FIG. 25

FIG. 26

EP 2 486 851 B1

FIG. 27

FIG. 28

FIG. 29

FIG. 30

EP 2 486 851 B1

EP 2 486 851 B1

600

**Measurement**

601 — Select A1c Protocol

601A — ☑ On the fly
estimate (default)

601B — ☒ Predict estimate

601C — ☒ component estimate

Accept        Decline

Additional screen(s)
to customize data
collection

602

FIG. 31

```
        ┌─────────────────┐
510 ─┐  │   COLLECT bG    │
     └─ │ MEASUREMENTS AND│─────────────┐              ┌ 500
        │ASSOCIATED CONTEXT│             │             ⟍
        └─────────────────┘              │
                │                        │
                ▼                        │
        ┌─────────────────┐              │
        │   WEIGHT THE bG │              │
520 ─┐  │   MEASUREMENTS  │              │
     └─ │    BASED ON THE │              │
        │ASSOCIATED CONTEXT│             │
        └─────────────────┘              │
```

SELECT TYPE(S) OF HbA1c VALUES TO DISPLAY

COMPARATIVELY DISPLAY SELECTED HbA1c VALUES

FIG. 32

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008125636 A
- US 5822715 A
- EP 0483595 A
- US 2008214841 A
- US 2010330598 A1
- US 20070010950 A1
- US 119143
- US 297733 A
- US 12119201 A

**Non-patent literature cited in the description**

- Primary Care Diabetes. ELSEVIER, 01 December 2008
- **MORTENSEN, H. B.** Glycated hemoglobin. Reaction and biokinetic studies. Clinical application of hemoglobin Alc in the assessment of metabolic control in children with diabetes mellitus,. *Danish medical bulletin,* 1985, vol. 32 (6), 309-328
- **NATHAN, D. M. ; SCHOENFELD, D. ; KUENEN, J. ; HEINE, R., J. ; BORG, R. ; ZHENG, H.** Translating the A1C Assay into estimated average glucose values. *Diabetes Care,* August 2008, vol. 31 (8), 1-6